# EUROPEAN PATENT SPECIFICATION

(11) **EP 3 868 378 B1**
(45) Date of publication and mention of the grant of the patent: **03.09.2025**
(21) Application number: 19874405.4
(22) Date of filing: 12.09.2019
(51) Int. Cl.: A61K 9/14, A61K 31/438, A61K 31/395, A61K 47/30, A61P 31/04

(54) **SOLID DISPERSION OF RIFAMYCIN-NITROIMIDAZOLE COUPLING MOLECULES AND USE THEREOF**
FESTE DISPERSION VON RIFAMYCIN-NITROIMIDAZOL-KOPPLUNGSMOLEKÜLEN UND IHRE VERWENDUNG
DISPERSION SOLIDE DE MOLÉCULES DE COUPLAGE DE RIFAMYCINE-NITROIMIDAZOLE ET SON UTILISATION

(30) Priority: 16.10.2018 CN 201811200947
(43) Date of publication of application: 25.08.2021
(73) Proprietor: TenNor Therapeutics (Suzhou) Limited, China (Jiangsu) Pilot Free Trade Zone (CN)
(72) Inventor: LIU, Yu, Suzhou, Jiangsu 215123 (CN); XU, Xiang Yi, Suzhou, Jiangsu 215123 (CN); MA, Zhenkun, Suzhou, Jiangsu 215123 (CN)
(74) Representative: Gong, Jinping
(86) International application number: PCT/CN2019/105617
(87) International publication number: WO 2020/078154

(56) References cited:
- WO-A1-2012/009387
- WO-A1-2018/157750
- WO-A2-2008/008480
- CN-A- 103 228 662
- CN-A- 106 420 633
- CN-A- 109 453 165

## Description

### BACKGROUND

### Technical Field

The present invention relates to a solid dispersion of a rifamycin-nitroimidazole coupling molecule and an application thereof, and belongs to the technical field of medicine.

Anaerobic and microaerobic infections are related to many common and major diseases. At present, a new antibacterial drug with unique mechanism of action has not been developed specifically for anaerobes/microaerobes in the world, and clinical application still relies on antibacterial drugs such as nitroimidazoles that have been on the market for more than 60 years. Because the problem of resistance to these drugs is severe, some common and major diseases have the trends of increased recurrence rates and decreased cure rates. Therefore, the development of new antibacterial drugs for anaerobe/microaerobe infection is an important clinical unmet need.

The rifamycin-nitroimidazole coupling molecule (as shown in formula I of this description) is a new multi-target antibacterial drug formed by the coupling of two pharmacophores of rifamycin and nitroimidazole through stable covalent bonds. It has potent antibacterial activity than the combination of two parent antibacterial drugs, has strong in vitro and in vivo antibacterial activity for anaerobes and microaerobes and particularly has obvious activity for drug-resistant bacteria. Compared with the current therapeutic drugs, the coupling molecule has obvious advantages in the aspects of reducing the development of drug resistance, sterilization speed, post-antibiotic effect and pharmacophore synergy. Safety pharmacological and toxicological studies show that the rifamycin-nitroimidazole coupling molecule has good safety tolerance. Therefore, the coupling molecule has application prospects in the aspects of treatment of diseases related to anaerobe/microaerobe infection, including Helicobacter pylori infection related digestive tract diseases, bacterial vaginosis and clostridium difficile infection related diarrhea. WO 2018/157750 describes an immediate release formulation of a rifamycin-nitroimidazole coupling molecule.

However, in the prior art, there is still a lack of preparations of the rifamycin-nitroimidazole coupling molecule with reliable curative effect.

### SUMMARY

In view of the above defects existing in the prior art, the purpose of the present invention is to provide a solid dispersion of a rifamycin-nitroimidazole coupling molecule as specified in any of claims 1-7 and an application as specified in claim 11 thereof. The solid dispersion of the rifamycin-nitroimidazole coupling molecule can be used as a formulation of a drug for treating microbial infection.

The purpose of the present invention is realized by the following technical solutions: A solid dispersion of a rifamycin-nitroimidazole coupling molecule comprises a rifamycin-nitroimidazole coupling molecule of a structure shown in formula I, a polymer carrier, functional excipient and a solvent.

The polymer carrier comprises one or a combination of more of hydroxypropyl cellulose-L (HPC-L), hydroxypropyl methylcellulose E3 (HPMC E3), polyvinyl caprolactam-polyvinyl acetate-polyethylene glycol graft copolymer (Soluplus) and polymethacrylate (Eudragit ^{®} EPO).

The functional excipient comprise one or a combination of more of sodium lauryl sulfate (SLS), meglumine, Vitamin E D -α-Tocopherol polyethylene glycol 1000 succinate (VE-TPGS) or TPGS and polysorbate 80 (Tween 80)

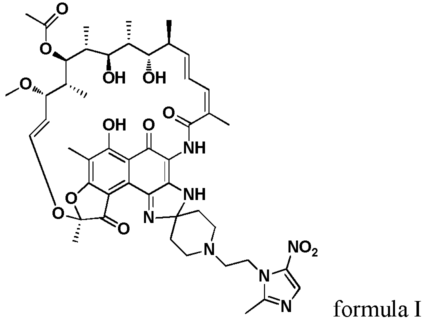

In the above solid dispersion, preferably, the polymethacrylate comprises, but is not limited to, Eudragit ^{®} EPO.

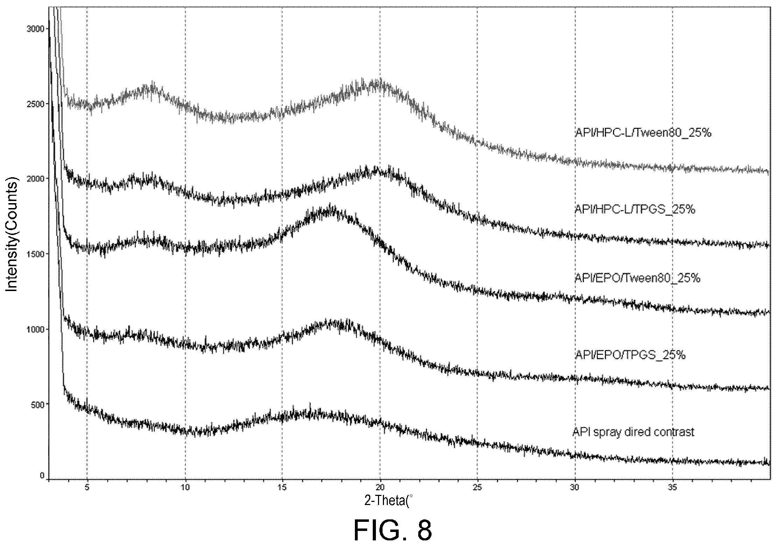

In the above solid dispersion, preferably, the solid dispersion is characterized by one or more XRPDs basically similar to Fig. 8.

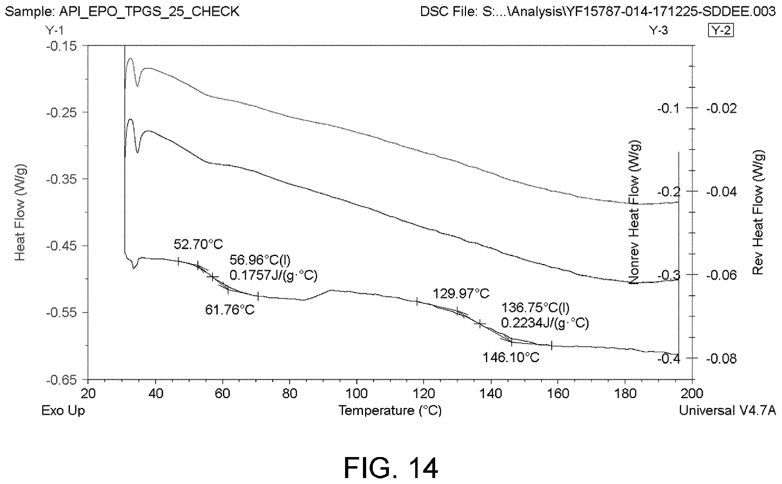

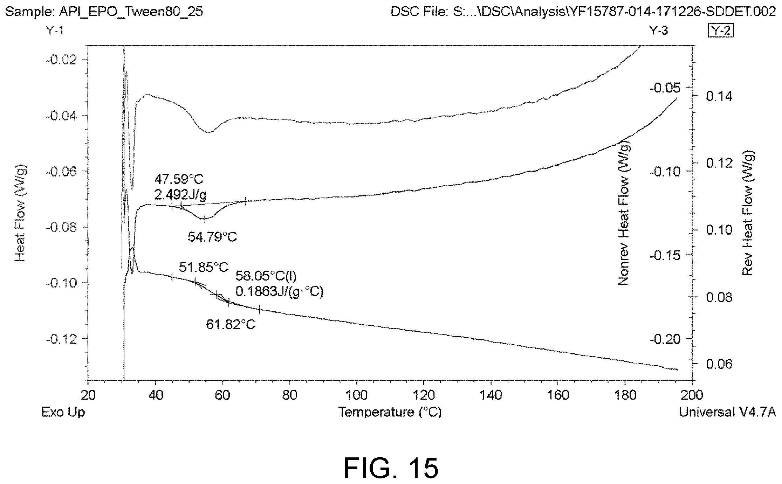

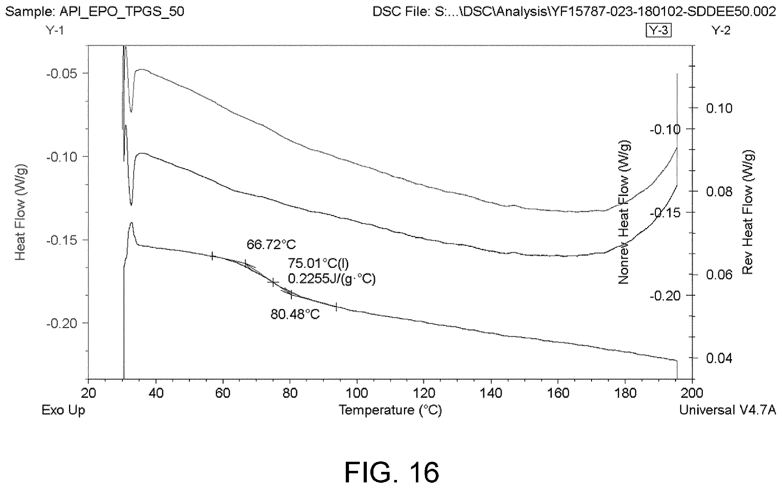

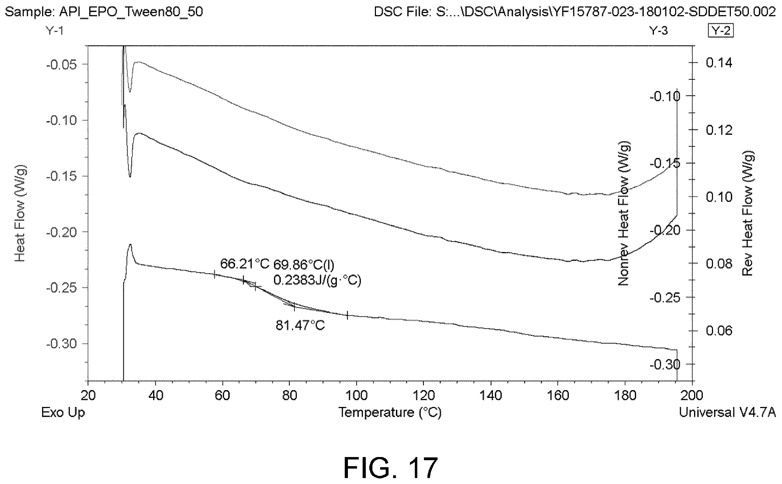

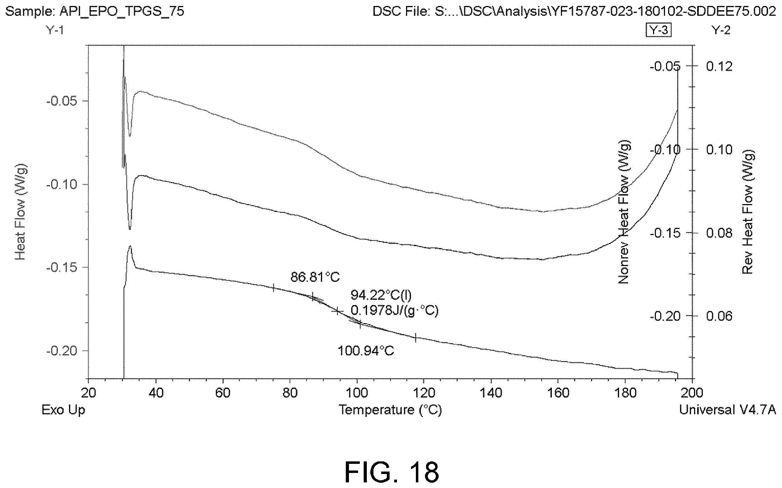

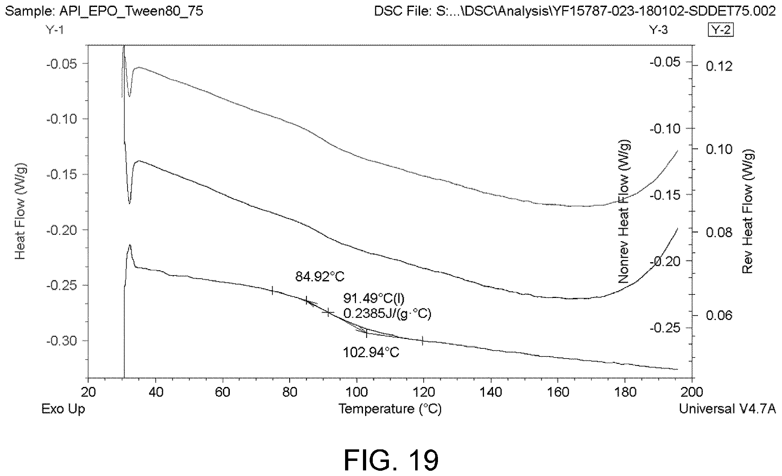

In the above solid dispersion, preferably, the solid dispersion is characterized by one or more thermograms basically similar to Fig. 14-Fig. 19.

In the above solid dispersion, preferably, the polymer carrier comprises HPC-L and/or Eudragit ^{®} EPO.

In the above solid dispersion, preferably, the functional excipient are VE-TPGS and/or Tween 80.

In the above solid dispersion, preferably, based on the total mass of the rifamycin-nitroimidazole coupling molecule, the polymer carrier and the functional excipient as 100%, the use amount of the rifamycin-nitroimidazole coupling molecule is 23.8%-71.2%, the use amount of the polymer carrier is 23.8%-71.2%, and the use amount of the functional excipient is 3%-7% (preferably 4%-6%).

In the above solid dispersion, the solvent can be a common reagent in the field. Preferably, the solvent comprises one or a combination of more of methanol, ethanol, acetone, ethyl acetate, tetrahydrofuran and dichloromethane.

In the above solid dispersion, preferably, when the solid dispersion is placed at 40°C /75%RH or 60°C/open conditions for 4 weeks, the solid dispersion remains amorphous according to XRPD.

The present invention also provides a granule as specified in claim 8 which comprises the above solid dispersion of the rifamycin-nitroimidazole coupling molecule.

The present invention also provides a pharmaceutical composition as specified in claim 9 which comprises the above granule.

The present invention also provides a pharmaceutical composition as specified in claim in claim 10 which comprises the above solid dispersion of the rifamycin-nitroimidazole coupling molecule.

The present invention also provides an application of the above solid dispersion of the rifamycin-nitroimidazole coupling molecule in preparation of a drug for treating diseases caused by microbial infection.

The present invention also provides an application of the above granule in preparation of a drug for treating diseases caused by microbial infection.

The present invention also provides an application of the above pharmaceutical composition in preparation of a drug for treating diseases caused by microbial infection.

The present invention has the following prominent effects:
The solid dispersion of the rifamycin-nitroimidazole coupling molecule of the present invention can be used as a formulation of a drug for treating microbial infection.

### BRIEF DESCRIPTION OF THE DRAWINGS

Fig. 1 is a chromatogram of a standard solution for content analysis of a dissolution experiment of a rifamycin-nitroimidazole coupling molecule in an embodiment.
Fig. 2 is a chromatogram of a standard solution for impurity analysis of a stability experiment of a rifamycin-nitroimidazole coupling molecule in an embodiment.
Fig. 3 is an ultraviolet absorption spectrum of a rifamycin-nitroimidazole coupling molecule in an embodiment.
Fig. 4 is a diagram of dissolution behaviors (25°C) of a solid dispersion film in simulated gastric fluid of pH 1.2.
Fig. 5 is a diagram of dissolution behaviors (25°C) of a solid dispersion film in acetate buffer salt of pH 4.5.
Fig. 6 shows comparison diagrams of polarized light microscopes of solid dispersion powder prepared by four prescription spray drying methods.
Fig. 7 shows scanning electron microscope comparison diagrams of solid dispersion powder prepared by the spray drying methods and raw material of a rifamycin-nitroimidazole coupling molecule.
Fig. 8 is an X-ray diffraction pattern of solid dispersion powder prepared by four prescription spray drying methods.
Fig. 9 is a diagram of dissolution behaviors of solid dispersion powder prepared by spray drying methods in acetate buffer of pH 4.5.
Fig. 10 shows comparison diagrams of polarized light microscopes of solid dispersions with different drug loadings in prescriptions 5-8.
Fig. 11 shows scanning electron microscope comparison diagrams of solid dispersion powder with different drug loadings in prescriptions 5-8.
Fig. 12 shows X-ray diffraction comparison diagrams of solid dispersion powder with different drug loadings in prescriptions 5-8.
Fig. 13 shows MDSC characterization curves of active pharmaceutical ingredient (API) of rifamycin-nitroimidazole coupling molecule powder.
Fig. 14 shows MDSC curves of solid dispersion powder of rifamycin-nitroimidazole coupling molecule (drug loading of 23.8%)/EPO/TPGS.
Fig. 15 shows MDSC curves of solid dispersion powder of rifamycin-nitroimidazole coupling molecule (drug loading of 23.8%)/EPO/Tween 80.
Fig. 16 shows MDSC curves of solid dispersion powder of rifamycin-nitroimidazole coupling molecule (drug loading of 47.6%)/EPO/TPGS.
Fig. 17 shows MDSC curves of solid dispersion powder of rifamycin-nitroimidazole coupling molecule (drug loading of 47.6%)/EPO/Tween 80.
Fig. 18 shows MDSC curves of solid dispersion powder of rifamycin-nitroimidazole coupling molecule (drug loading of 71.4%)/EPO/TPGS.
Fig. 19 shows MDSC curves of solid dispersion powder of rifamycin-nitroimidazole coupling molecule (drug loading of 71.4%)/EPO/Tween 80.
Fig. 20 is a diagram of dissolution behaviors of solid dispersion powder with different drug loadings in acetate buffer of pH 4.5.
Fig. 21 shows MDSC curves of a 5-day stability experiment of a rifamycin-nitroimidazole coupling molecule (23.8%)/EPO/TPGS at 50°C/open conditions.
Fig. 22 shows MDSC curves of a 5-day stability experiment of a rifamycin-nitroimidazole coupling molecule (23.8%)/EPO/Tween 80 at 50°C/open conditions.
Fig. 23 shows MDSC curves of a 5-day stability experiment of a rifamycin-nitroimidazole coupling molecule (47.6%)/EPO/TPGS at 50°C/open conditions.
Fig. 24 shows MDSC curves of a 5-day stability experiment of a rifamycin-nitroimidazole coupling molecule (47.6%)/EPO/Tween 80 at 50°C/open conditions.
Fig. 25 shows MDSC curves of a 5-day stability experiment of a rifamycin-nitroimidazole coupling molecule (71.4%)/EPO/TPGS at 50°C/open conditions.
Fig. 26 shows MDSC curves of a 5-day stability experiment of a rifamycin-nitroimidazole coupling molecule (71.4%)/EPO/Tween 80 at 50°C/open conditions.
Fig. 27 shows MDSC curves of a 5-day stability experiment of a rifamycin-nitroimidazole coupling molecule (23.8%)/EPO/TPGS at 40°C/75%RH conditions.
Fig. 28 shows MDSC curves of a 5-day stability experiment of a rifamycin-nitroimidazole coupling molecule (23.8%)/EPO/Tween 80 at 40 °C /75%RH conditions.
Fig. 29 shows MDSC curves of a 5-day stability experiment of a rifamycin-nitroimidazole coupling molecule (47.6%)/EPO/TPGS at 40°C/75%RH conditions.
Fig. 30 shows MDSC curves of a 5-day stability experiment of a rifamycin-nitroimidazole coupling molecule (47.6%)/EPO/Tween 80 at 40 °C /75%RH conditions.
Fig. 31 shows MDSC curves of a 5-day stability experiment of a rifamycin-nitroimidazole coupling molecule (71.4%)/EPO/TPGS at 40°C/75%RH conditions.
Fig. 32 shows MDSC curves of a 5-day stability experiment of a rifamycin-nitroimidazole coupling molecule (71.4%)/EPO/Tween 80 at 40 °C /75%RH conditions.
Fig. 33 is an XRPD diagram of a solid dispersion obtained in prescription 3 under different stability conditions for 2 weeks.
Fig. 34 is an XRPD diagram of a solid dispersion obtained in prescription 3 under different stability conditions for 4 weeks.
Fig. 35 shows MDSC curves of a solid dispersion obtained in prescription 3 under 25°C /60%RH conditions for 2 weeks.
Fig. 36 shows MDSC curves of a solid dispersion obtained in prescription 3 under 40°C /75%RH conditions for 2 weeks.
Fig. 37 shows MDSC curves of a solid dispersion obtained in prescription 3 at 60°C for 2 weeks.
Fig. 38 shows MDSC curves of a solid dispersion obtained in prescription 3 under 25°C /60%RH conditions for 4 weeks.
Fig. 39 shows MDSC curves of a solid dispersion obtained in prescription 3 under 40°C /75% conditions for 4 weeks.
Fig. 40 shows MDSC curves of a solid dispersion obtained in prescription 3 at 60°C for 4 weeks.
Fig. 41 shows dissolution curves (37°C) of capsules of a rifamycin-nitroimidazole coupling molecule (specification of 100 mg) at pH 4.5.

### DESCRIPTION OF THE EMBODIMENTS

To understand the technical features, purpose and beneficial effects of the present invention more clearly, the technical solutions of the present invention are described in detail below, but shall not be understood as the limitation of the implementation scope of the present invention. The experimental methods described in the following embodiments are conventional methods unless otherwise specified; and the reagents and materials are commercially available unless otherwise specified.

The "analogous, similar" used in this description, when presenting the forms of characteristics similar to, for example, XRPD, IR, Raman spectroscopy, DSC, TGA, NMR and SSNMR, mean that polymorphs or co-crystals can be identified through the method and can be in a similar to basically similar range as long as the material is identified by the method as having the variability expected by those skilled in the art (according to experimental changes including, for example, the instrument used, time of day, humidity, season, pressure, room temperature, and the like).

The polymorphy used in this description refers to the appearance of different crystal forms of a single compound in different hydration states (such as the properties of some compounds and complexes). Therefore, the polymorphs are different solids that share the same molecular formula, but each polymorph may have unique physical properties. Therefore, the single compound may produce multiple polymorphs, and each form has different and unique physical properties, such as solubility curve, melting point temperature, hygroscopicity, particle shape, density, fluidity, compactability and/or x-ray diffraction peaks. The solubility of each polymorph can be changed, so identification of the presence of a pharmaceutical polymorph is necessary to provide a drug with a predictable solubility curve. It is desirable to investigate all solid-state forms of drugs including all the polymorphs and determine the stability, dissolution and fluidity of each polymorph. The polymorphs of the compounds can be distinguished in a laboratory through X-ray diffraction spectroscopy and other methods such as infrared spectroscopy.

The materials and equipment conditions used in the embodiments of this description, as well as some involved methods are as follows:

**Table 1 Reagent**

| Name | Level | Supplier | Batch Number |
|---|---|---|---|
| Ultra-pure Water | Chromatographic Grade | Homemade | N/A |
| Acetonitrile | Chromatographic Grade | Merck | JA059130 |
| Trifluoroacetic Acid | Chromatographic Grade | J&K Chemicals | LN20M33 |
| Methanol | Chromatographic Grade | Merck | 10899607729 |
| Ethanol | Chromatographic Grade | J.T.Baker | 0000157575 |
| Acetone | Analytical Grade | CINC | 7306090 |
| Ethyl Acetate | Analytical Grade | CINC | 093060901 |
| Tetrahydrofuran | Chromatographic Grade | Macron | 1613729801 |
| Dichloromethane | Chromatographic Grade | CNW Technologies | M1130432 |
| Hydrochloric Acid | Analytical Grade | Shanghai Bohr Chemical Reagent | 20171031 |
| Simulated Gastric Fluid of pH 1.8 | N/A | Homemade | N/A |
| Simulated Gastric Fluid of pH 1.2 | N/A | Homemade | N/A |
| Sodium Hydroxide | Analytical Grade | Alfa Aesar | 10178067 |
| Acetic Acid | Chromatographic Grade | Sigma | ST8D9613V |
| Ammonium Acetate | Chromatographic Grade | Fluka | BCBR4575V |

**Table 2 Excipient**

| Name | Supplier | Batch Number |
|---|---|---|
| PVP K29/32 | Ashland | 0001910711 |
| PVP VA64 | BASF | 13968068E0 |
| HPC-L | Tosoh | NCA-2021 |
| HPMC E3 | Colorcon | PD280165 |
| Hydroxypropyl Methylcellulose-M | Shin-Etsu | 9043106 |
| Hydroxypropyl Methylcellulose-H | Shin-Etsu | 13050001 |
| Soluplus | BASF | 84414368E0 |
| Eudragit EPO | Evonik | G120231025 |
| Sodium Lauryl Sulfate | BASF | 0008985310 |
| Meglumine | Alfa Aesar | 10151696 |
| VE-TPGS | Healthcare | C071710801 |
| Tween 80 | Sigma | 172402112 |

**Table 3 Instrument and Equipment**

| Name | Type | Supplier |
|---|---|---|
| Balance | XS205Dualrange | Mettler |
| Balance | CPA 225D | Sartorius |
| Balance | XP6 | Mettler |
| Polarized Light Microscope | LV100POL Eclipse | Nikon |
| Scanning Electron Microscope | ProX | Phenom |
| X-ray diffractometer | D8 Advance | Bruker |
| Differential Calorimeter | Q2000 | TA Instrument |
| Thermal Gravimetric Analyzer | Q5000 IR | TA Instrument |
| Dynamic Vapor Sorption | Advantage-1 | SMS |
| Ultra-pure Water Purifier | Milli-Q Direct 8 | Millipore |
| pH Meter | SevenMulti | Mettler |
| Turbine Mixer | H-101 | Kang |
| Constant Temperature Suspension Instrument | YQH-0623 | Eppendorf |
| Magnetic Stirrer | 98-1 | Shanghai Xerox |
| Magnetic Stirrer | RCT basic | IKA |
| High Performance Liquid Chromatography | Infinity 1200 | Agilent |
| High Performance Liquid Chromatography | Infinity 1260 | Agilent |
| Centrifuge | H1650 | Hunan Xiangyi |
| Centrifuge | Centrifuge5418 | Eppendorf |
| Ultrasonic Cleaner | KQ-250DB | Kunshan Instrument |
| Water Bath Device | HHS | Buchi |
| Air Dry Oven | DZF-6050 | Boxun |
| Spray Dryer | B-90 HP | Buchi |
| Spray Dryer | 4M8-Trix | Procept |
| 50°C Air Dry Oven | GZX-9140MBE | Boxun |
| 60°C Air Dry Oven | GZX-9140MBE | Boxun |
| 40°C/75%RH Stability Chamber | KBF720 | BINDER |
| 25°C/60%RH Stability Chamber | KBF240 | BINDER |
| Tap Density Tester | SVM203 | ERWEKA |

Instrument and equipment parameters:
Polarized light microscope (PLM)
Nikon LV100POL
Objective amplification: 10x/20x/50x
X-ray powder diffractometer (XRPD)

About 5 mg of samples are laid on a monocrystalline silicon plate, and a test method is performed as follows:
Ray tube: Cu: K-Alpha (λ=1.54179Ǻ);
Generator: Voltage: 40 kV; Current: 40 mA;
Scanning angle: 3 to 40 deg;
Sample rotation speed: 0 rpm;
Scanning speed: 10 deg./min.

Differential scanning calorimeter (DSC)
Standard method: heating range: 30°C to 300°C; heating rate: 10°C/min;
Modulated differential calorimetry: heating range: 0°C/30°C to 200°C; heating rate: 2°C/min;
adjusting range: ±1°C; adjusting period: 60s.

Thermal gravimetric analyzer (TGA)
Heating range: ambient temperature: -300°C; heating rate: 10°C/min.

Dynamic vapor sorption (DVS)
10-20 mg of samples are put into a DVS sample disk at a test temperature of 25°C.

Humidity cycle parameters are set as follows: balance condition: weight %/ min <0.01% (each balance time: minimum 10 minutes and maximum 180 minutes); predrying: 0%RH balance for 2 hours; Humidity balance setting: 0, 10, 20, 30, 40, 50, 60, 70, 80, 90, 80, 70, 60, 50, 40, 30, 20, 10 and 0.

Scanning electron microscope (SEM)
Voltage: 10KV; current: Point; mode: BSC Full.

Bulk/tap density measurement
Equipment: 10 ml measuring cylinder;
Vibration height: 14 mm;
Vibration frequency: 300 times/min.

The sample is sieved (1.0 mm, No. 18 screen) into the 10 ml measuring cylinder. An initial volume is recorded; and the tap density is measured according to USP as follows: the measuring cylinder is vibrated for 500 times and the volume, Va is measured. The measuring cylinder is vibrated again for 750 times and the volume, Vb is measured. If the difference between the two volumes is less than 2%, Vb is the final tap volume. If the volume change is greater than 2%, the operation is repeated in the form of 1250 vibrations each time until the change of two adjacent volumes is less than 2%, and the last volume is the final tap volume.

Preparation of simulated gastric fluid of pH 1.2: 400 µL of 12N concentrated hydrochloric acid is absorbed by a pipette to 100 mL of simulated gastric fluid of pH 1.8. The pH of the solution is detected by a pH meter as 1.21.

Simulated gastric fluid (pH 1.8): 950 mL of ultra-pure water is measured into a 1L volumetric flask, and 1.4 mL of 12N concentrated hydrochloric acid and 2g of sodium chloride are added and stirred uniformly. The volume is fixed by the ultra-pure water.

Preparation of acetate buffer of pH 4.5

869.05 mg of solid sodium hydroxide particles are weighed; 1L of ultra-pure water is measured into a 1L glass bottle; 3.15 mL of glacial acetic acid is taken by the pipette into the bottle; and the pH of the solution is detected by the pH meter as 4.53.

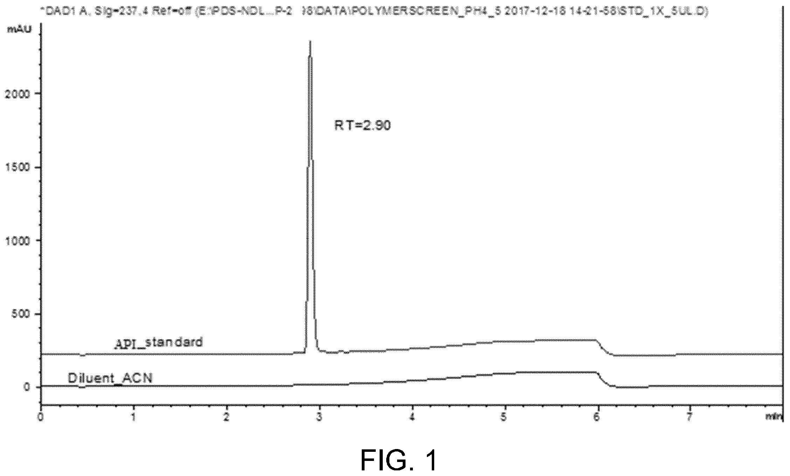

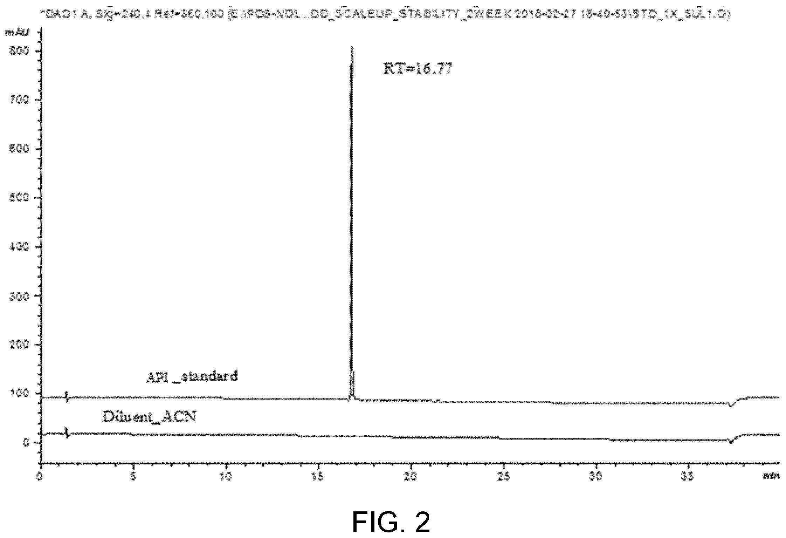

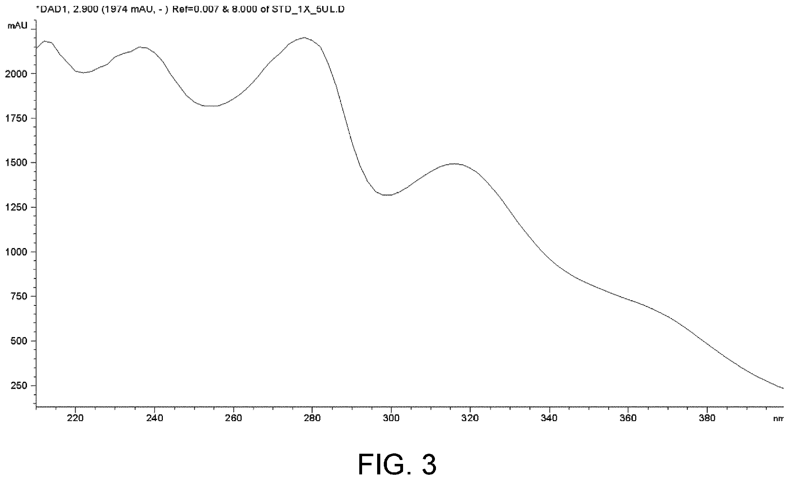

High performance liquid chromatography (HPLC) determination method of the rifamycin-nitroimidazole coupling molecule: HPLC content and impurity detection methods are shown in Table 4 and Table 5. The UV absorption spectrum of the rifamycin-nitroimidazole coupling molecule is shown in Fig. 3, and the standard solution chromatograms are shown in Fig. 1 and Fig. 2. In the two HPLC methods, the retention time of the rifamycin-nitroimidazole coupling molecule is 2.90 minutes and 16.77 minutes, respectively. System suitability verification and linear interval results are shown in Table 6 and Table 7. The two methods are respectively used for content determination in the dissolution experiment, and the determination of the recovery rate and relevant impurities in the stability experiment.

**Table 4 HPLC Method for Content Analysis of Dissolution Experiment**

| Instrument | Agilent 1200 HPLC | | |
|---|---|---|---|
| Chromatographic Column | Waters xBridge C18, 4.6*50mm 3.5-Micron | | |
| Column Temperature | 40°C | | |
| Mobile Phase Gradient Procedure | Time (Minute) | A: 0.1% Trifluoroacetic Acid Water | B: 0.1% Acetonitrile Trifluoroacetate |
| | 0.00 | 85 | *15* |
| | 4 | 5 | 95 |
| | 5 | 5 | 95 |
| | 5.1 | 85 | *15* |
| | 8 | 85 | *15* |
| Flow Velocity | 1.5 ml/min | | |
| Wavelength | 237 nm | | |
| Sampling Volume | 5 µL | | |
| Diluent | Acetonitrile | | |
| Integral Interval | 2-4 min | | |

**Table 5 HPLC Method for Impurity Analysis of Stability Experiment**

| Instrument | Agilent 1260 HPLC | | |
|---|---|---|---|
| Chromatographic Column | HALO C18,150 mm*4.6 µm, 2.7 µm | | |
| Column Temperature | 30°C | | |

| | Time (Minute) | A: 10mM Ammonium Acetate Water (pH=4.3) | B: Acetonitrile |
|---|---|---|---|
| Mobile Phase Gradient Procedure | 0.0 | 75 | 25 |
| | 18.0 | 35 | 65 |
| | 30.0 | 20 | 80 |
| | 35.0 | 20 | 80 |
| | 35.1 | 75 | 25 |
| | 40 | 75 | 25 |
| Flow Velocity | 1.0 ml/min | | |
| Wavelength | 240 nm | | |
| Sampling Volume | 5 µL | | |
| Diluent | Acetonitrile | | |
| Integral Interval | 4-21 min | | |

**Table 6 System Suitability by HPLC Method for Content Analysis of Dissolution Experiment**

| Detection Items | Standard | Result |
|---|---|---|
| Baseline Impurity | No obvious impurity interference (<limit of quantitation) | Passed |
| Sensitivity | Signal-to-noise ratio ≥ 10 | Passed |
| Total Related Impurity | N/A | 0.5% |
| Recovery Rate | 98.0%-102.0% | 99.7% |
| Degree of Separation | ≥ 1.2 | 1.2 |
| USP Tailing Factor | 0.8 ≤ T ≤ 2.0 | 1.1 |
| repeatability | ≤ 2.0% | 0.99% |
| Linear Range (mg/ml) | R²≥ 0.9990 | 0.9999 |

**Table 7 System Suitability by HPLC Method for Related Substance Analysis of Stability Experiment**

| Detection Items | Standard | Result |
|---|---|---|
| Baseline Impurity | No obvious impurity interference (<limit of quantitation) | 21.46 min |
| Sensitivity | Signal-to-noise ratio ≥ 10 | Passed |
| Total Related Impurity | N/A | 1.1% |
| Recovery Rate | 98.0%-102.0% | 101.1% |
| Degree of Separation | ≥ 1.2 | 2.4 |
| USP Tailing Factor | 0.8 ≤ T ≤ 2.0 | 1.1 |
| repeatability | ≤ 2.0% | 0.10% |
| Linear Range (mg/ml) | R²≥ 0.9990 | 0.99999 |

### Embodiments

This embodiment provides a solid dispersion of a rifamycin-nitroimidazole coupling molecule, comprising a rifamycin-nitroimidazole coupling molecule of a structure shown in formula I, a polymer carrier, functional excipient and a solvent.

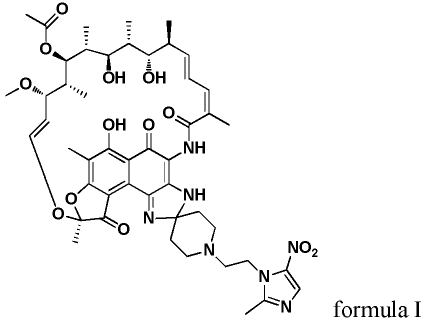

The rifamycin-nitroimidazole coupling molecule of this embodiment (as shown in formula I, abbreviated as API or crude drug for convenience of statement) is produced by TenNor Therapeutics (Suzhou) Ltd.

In this embodiment, the solvent is firstly screened and determined: the solvent comprises one or a combination of more of methanol, ethanol, acetone, ethyl acetate, tetrahydrofuran and dichloromethane. About 10 mg of rifamycin-nitroimidazole coupling molecules are weighed into a 1.5 mL liquid phase bottle, and appropriate volumes of different solvents are added respectively to observe and judge whether the coupling molecule is completely dissolved and judge approximate solubility of the coupling molecule.

Results are shown in Table 8 below. The results show that the rifamycin-nitroimidazole coupling molecule is easily soluble in various common spray drying solvents (> 100 mg/mL). Moreover, the permitted daily exposure levels (PDE levels) of organic solvents prescribed by ICH are listed for reference.

**Table 8 Approximate Solubility of Rifamycin-Nitroimidazole Coupling Molecule in Different Solvents**

| Solvent | PDE Level | Approximate Solubility (mg/mL) |
|---|---|---|
| Methanol | 2 | > 300 |
| Ethanol | 3 | 167-200 |
| Acetone | 3 | > 300 |
| Ethyl Acetate | 3 | 167-200 |
| Tetrahydrofuran | 2 | > 200 |
| Dichloromethane | 2 | > 300 |

In this embodiment, polymer carriers are screened:
In order to determine suitable quick-release carrier excipient, six polymer carriers, i.e., polyvinylpyrrolidone K30 (PVP K30), polyvinylpyrrolidone VA64 (PVP-VA64), hydroxypropyl cellulose L (HPC-L), hydroxypropyl methylcellulose E3 (HPMC E3), polyethylene caprolactam-polyvinyl acetate-polyethylene glycol graft copolymer (Soluplus) ^{®} and polymethacrylate (Eudragit ^{®} EPO), are respectively used as carrier excipient, to prepare binary solid dispersions by a solvent volatilization method and assess dissolution behaviors thereof by a small-bottle dissolution experiment.

Preparation of binary solid dispersion (ASD for short): About 25 mg of rifaomycin-nitroimidazole coupling molecules and about 25 mg of carrier excipient are weighed, and placed into the same 40mL glass bottle. The glass bottle is shaken after the addition of solvent, so as to achieve complete dissolution, as shown in Table 9. The open 40 mL glass bottle is covered with perforated aluminum-foil paper to prevent cross contamination and placed into a 35°C vacuum drying oven for rapid volatilization. After 40 hours of vacuum and full drying, the binary dispersion forms a purple film on the bottom of the bottle to obtain a binary solid dispersion film.

**Table 9 Preparation of Binary Solid Dispersion Film**

| Solid Dispersion Composition | Solvent | Preparation Process | Physical Appearance |
|---|---|---|---|
| API: polyvidone K30 = 1:1 | Acetone: dichloromethane = 1:1, 2 mL | Vibration, vacuum | Purple film |
| API: polyvidone VA64 = 1:1 | Dichloromethane, 1 mL | Vibration, vacuum | Purple film |
| API: hydroxypropyl cellulose L = 1:1 | Dichloromethane, 1 mL | Vibration, vacuum | Purple film |
| API: hydroxypropyl methylcellulose E3 = 1:1 | Methanol, 2 mL | Water bath for 5 minutes at 50 °C, ultransonic, vacuum | Purple film |
| API: Soluplus = 1:1 | Dichloromethane, 1 mL | Vibration, vacuum | Purple film |
| API: Eudragit EPO = 1:1 | Dichloromethane, 1 mL | Vibration, vacuum | Purple film |
| API Control | Dichloromethane, 1 mL | Vibration, vacuum | Purple film |

The prepared binary solid dispersion film is subjected to dissolution experiments:
In the 40 mL glass bottle containing solid dispersion films, 0.5 mL of simulated gastric fluid of pH 1.2 and 1 mL of acetate buffer of pH 4.5 are respectively taken as dissolution media (25°C) (the target concentration is 50 mg ml), to seal the glass bottle and complete the vortex processing for 5 seconds; 100 µL of liquid is taken as an initial sampling point (0 minute) and then the glass bottle is shaken by a rocking bed; 100 µL of solution is taken into centrifugal tubes respectively after 10 minutes, 30 minutes, and 60 minutes, to perform the centrifugation at a rotational speed of 14000 rpm for 5 minutes; and the supernatant is taken into a small liquid phase bottle and is diluted with the acetonitrile to a suitable multiple, and high-performance liquid chromatograph is taken into the glass bottle, to record a chromatogram map and calculate the concentration according to the peak area by an external standard method.

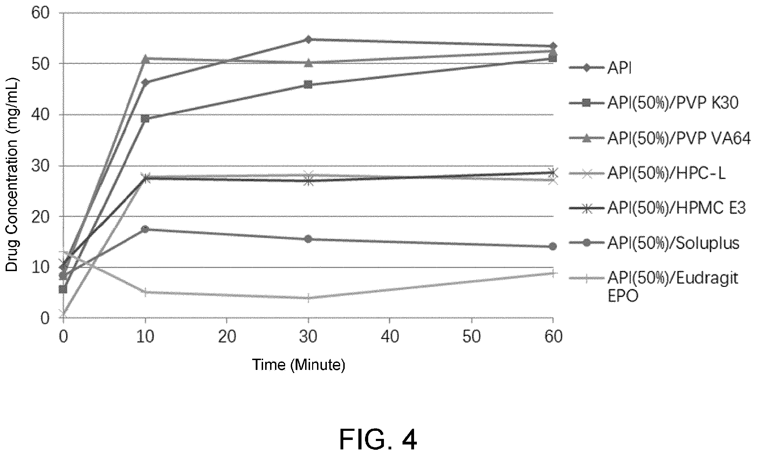

Dissolution results of rifamycin-nitroimidazole coupling molecular solid dispersions taking different excipient as carriers in the simulated gastric fluid media of pH 1.2 are shown in Table 10 and Figure 4. The results show that all solid dispersion films except HPC-L, including API film control, indicate a rapid drug release effect of 5-13 mg/mL after 5-second vortex processing. Within 10 to 60 minutes, only the systems taking PVP K30 and PVP-VA64 as carriers indicate the same release rate and the same release degree as the API film control, while the systems taking HPC-L, HPMC E3, Soluplus ^{®} and Eudragit ^{®} EPO as carriers indicate half or less of the release degree than API films. The reason is that PVP K30 and PVP-VA64 are carrier excipient with high wettability, which can dissolve and release drugs in the dissolution media, while HPC-L, HPMC and Soluplus ^{®} are relatively low in hydrophilicity, which are not conducive to drug release. In addition, Eudragit ^{®} EPO also shows the extremely high rapid release capacity at the initial point, reaching up to 13 mg/mL, but then dropping to 5 mg/mL. According to the characteristics of Eudragit ^{®} EPO excipient, the continuous dissolution of Eudragit ^{®} EPO increases the pH value to 6.5 (1 hour). But, according to the software prediction, the pKa of drugs is about 2.7. The pH change of the systems result in the balanced movement of the API dissociation and the decreased dissociation degree. Therefore, in order to design the experiments more reasonably, the influence of Eudragit ^{®} EPO concentration on the pH of the dissolution media is also studied in the subsequent experiments.

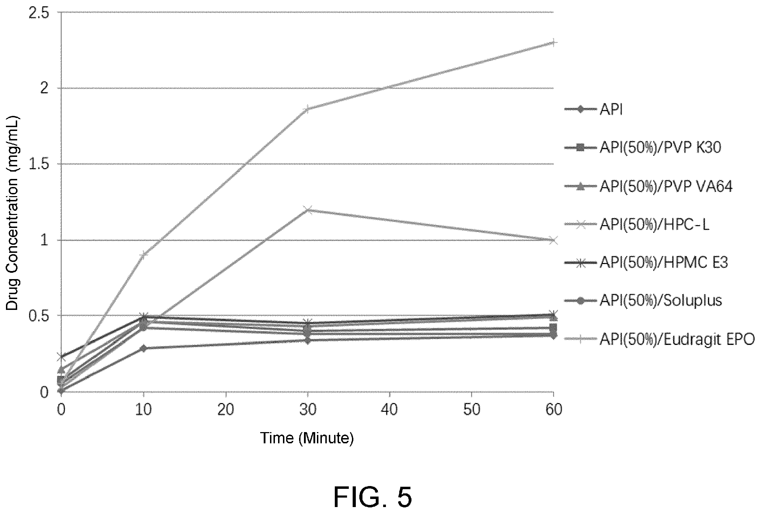

Dissolution experiment of acetate buffer of pH 4.5: A rifamycin-nitroimidazole coupling molecule is dissociated less and has higher hydrophobicity in the acetate buffer of pH 4.5 because pKa is about 2.7. Therefore, compared with the simulated gastric fluid of pH 1.2, the dissolution rate and the dissolution degree are significantly reduced. The dissolution results are shown in Table 11 and Figure 5. The results show that the films taking Eudragit ^{®} EPO and HPC-L
as carriers can significantly improve the drug dissolution rate at the system of pH 4.5, and can effectively maintain the release degree for 60 minutes with 6 times and 3 times of API film control, respectively. In addition, from the experimental phenomenon, the dissolved liquid of the solid dispersion film taking HPC-L as a carrier indicates an uniform and stable suspension state, so Eudragit ^{®}
EPO and HPC-L are chosen as carrier matrixes for the next step of spray drying dispersion research.

**Table 10 Dissolution Results of Solid Dispersion Film in Simulated Gastric Fluid of pH 1.2 (25°C)**

| Solid Dispersion Film | Drug Concentration (mg/mL) | | | | pH | Phenomenon |
|---|---|---|---|---|---|---|
| | 0 min | 10 min | 30 min | 60 min | | |
| API Control | 10.0 | 46.4 | 54.7 | 53.5 | 2.0 | Clear |
| API: PVP K30 = 1:1 | 5.6 | 39.2 | 45.9 | 51.0 | 1.8 | Clear |
| API: PVP VA64 = 1:1 | 8.4 | 51.0 | 50.3 | 52.5 | 1.7 | Clear |
| API: HPC-L = 1:1 | 0.9 | 27.8 | 28.1 | 27.2 | 1.9 | Suspension |
| API: HPMC E3 = 1:1 | 10.8 | 27.5 | 27.0 | 28.6 | 1.6 | Suspension |
| API: Soluplus = 1:1 | 8.3 | 17.4 | 15.5 | 14.1 | 1.8 | Suspension |
| API: Eudragit EPO = 1:1 | 13.0 | 5.1 | 4.0 | 8.8 | 6.5 | Suspension |

**Table 11 Dissolution Behavior of Solid Dispersion Film in Acetate Buffer Medium of pH 4.5**

| Solid Dispersion Film | Drug Concentration (mg/mL) | | | | pH | Phenomenon |
|---|---|---|---|---|---|---|
| | 0 min | 10 min | 30 min | 60 min | | |
| API Control | 0.01 | 0.29 | 0.34 | 0.37 | 4.5 | Clear solution, incomplete dissolved film |
| API: PVP K30 = | 0.08 | 0.46 | 0.40 | 0.42 | 4.5 | Suspension, incomplete dissolved film |
| API: PVP VA64 = 1:1 | 0.15 | 0.46 | 0.43 | 0.49 | 4.4 | Suspension, incomplete dissolved film |
| API: HPC-L = 1:1 | 0.03 | - | 1.2 | 1.0 | 4.4 | Homogeneous suspension, incomplete dissolved film |
| API: HPMC E3 = 1:1 | 0.23 | 0.49 | 0.45 | 0.51 | 4.5 | Suspension, incomplete dissolved film |
| API: Soluplus = 1:1 | 0.06 | 0.42 | 0.38 | 0.38 | 4.5 | Suspension, incomplete dissolved film |
| API: Eudragit EPO = 1:1 | 0.06 | 0.90 | 1.86 | 2.3 | 5.3 | Suspension, incomplete dissolved film |

Effect of Eudragit ^{®} EPO on pH and solubility of solution

In order to further research the gradually decreased behavior of the dissolution concentration of rifamycin - nitroimidazole coupling molecule in simulated gastric fluid of pH = 1.2 (shown in Table 10), the pH value - concentration dependence of the Eudragit ^{®} EPO solution is measured by configuring different concentrations of Eudragit ^{®} EPO, and the results are shown in Table 12. In addition, the solid dispersions of rifamycin - nitroimidazole coupling molecule/EPO(10/3) are prepared and the dissolution experiment is carried out, verifying the effect of EPO contents on the dissolution results, and the results are shown in Table 13.

The results further confirm that when the concentration of the Eudragit ^{®} EPO is increased to 25 mg/mL, the pH value of the solution is increased to 2.57, hereafter, the pH value of the solution is gradually increased with the dissolution of the Eudragit ^{®} EPO, and at the same time, the solubility of the rifamycin - nitroimidazole coupling molecule decreases rapidly as the pH of the dissolution medium increases. The results indicate that the effect of the Eudragit ^{®} EPO in a dissolution system on the pH plays an important role in the dissolution behavior of the formulation, and should be carefully considered in the future prescription design.

**Table 12 pH - Concentration Relationship of Eudragit ^{®} EPO Solution (25°C)**

| | | | | | |
|---|---|---|---|---|---|
| EPO Concentration (mg/mL) | 0 | 5 | 10 | 25 | 50 |
| pH Value | 1.2 | 0.95 | 1.09 | 2.57 | 6.47 |

**Table 13 Dissolution Results of Solid Dispersion of Rifamycin - Nitroimidazole Coupling Molecule/EPO(10/3)(37°C)**

| Time | 0 min | 10 min | 30 min | 60 min |
|---|---|---|---|---|
| Concentration (mg/mL) | 17.6 | 24.6 | 10.2 | 8.0 |
| pH value | 1.45 | 2.67 | 3.09 | 3.15 |

In this embodiment, the functional excipient are screened.

In order to further increase the dissolution performance of the rifamycin - nitroimidazole coupling molecule, sodium dodecyl sulfate, meglumine, VE-TPGS or Tween 80 is respectively added in the simulated gastric fluid system of pH=1.2 or the acetate buffer system of pH=4.5,to prepare the dissolution medium containing 3 mg/mL of functional excipient. About 100 mg (or 20 mg) of rifamycin - nitroimidazole coupling molecules are weighed and put into a 1.5 ml high-performance liquid phase bottle, 1 ml of simulated gastric fluid of pH 1.2 (or acetate buffer of pH 4.5) containing functional excipient is added, and the rifamycin - nitroimidazole coupling molecules are put into a constant temperature mixing instrument at 37°C, the rotation speed is 700 rpm, and after 120 minutes, 500 µl of liquid is taken into a centrifuge tube and centrifuged at 14,000 rpm for 5 min. The supernatant liquid is centrifuged again in the same condition, and the supernatant liquid after twice centrifugation is taken into the liquid phase bottle, and diluted to a proper amount of times with acetonitrile as the solution to be tested, a high performance liquid chromatograph is injected to record a chromatogram, and the concentration is calculated by a peak area using an external standard method.

The results indicate that in either simulated gastric fluid of pH 1.2 or acetate buffer of pH 4.5, VE-TPGS and Tween-80 can increase the two-hour dissolution rate of the rifamycin - nitroimidazole coupling molecule, and especially in the pH 4.5 system, compared to an API powder control, the VE-TPGS and Tween-80 can respectively increase the two-hour dissolution rate to 6 times and 4 times.

**Table 14 Two-hour Solubility of Rifamycin - Nitroimidazole Coupling Molecule in Functional Excipient Solution**

| Dissolution System | Functional Excipient | Initial pH | Final pH | Two-hour Dissolution Rate | Physical Appearance |
|---|---|---|---|---|---|
| 100 mg of API + 1 mL of Simulated Gastric Fluid of pH 1.2 | - | 1.20 | 2.5 | 9.8 | Suspension |
| | SLS | 1.21 | 2.72 | 6.7 | Suspension |
| | VE-TPGS | 1.22 | 2.60 | 11.5 | Suspension |
| | Tween80 | 1.22 | 2.60 | 11.7 | Suspension |
| 20 mg of API + 1 mL of Acetate Buffer of pH | - | 4.53 | 4.45 | 0.15 | Suspension |
| | SLS | 4.57 | 4.65 | 0.06 | Suspension |
| 4.5 | Meglumine | 5.02 | 4.91 | 0.10 | Suspension |
| | VE-TPGS | 4.57 | 4.45 | 0.84 | Suspension |
| | Tween80 | 4.57 | 4.46 | 0.61 | Suspension |

In this embodiment, the solid dispersion is prepared by a spray drying method: According to the dissolution behavior and the suspension state of solid dispersions in dissolution medium of pH 4.5 in a screening experiment of the excipient, Eudragit^{®} EPO and HPC-L are selected as carriers for spray drying. At the same time, in order to better improve the dissolution behavior of the formulation, VE TPGS or Tween 80 is respectively added to different solid dispersions as functional excipient. The solid dispersion components are shown in Table 15, the acetone is used as spray drying solvent, and the technological parameters are shown in Table 16. The solid dispersions prepared by spray drying are dried in a 35°C vacuum drying oven for 40 hrs and stored in a -20°C refrigerator.

**Table 15 Composition of Solid Dispersion Components**

| No. | Components | |
|---|---|---|
| | API + Excipient + Surfactant | Mass (mg) 23.8% + 71.2% + 5.0% |
| Prescription 1 | API + Eudragit EPO + TPGS | 600 + 1800 + 126 |
| Prescription 2 | API + HPC-L + TPGS | 600 + 1800 + 126 |
| Prescription 3 | API + Eudragit EPO + Tween-80 | 400 + 1200 + 84 |
| Prescription 4 | API + HPC-L + Tween-80 | 400 + 1200 + 84 |
| - | Spray Drying Control of API | 300 |

**Table 16 Spray Drying Technological Parameters**

| Item | Parameter | | | | |
|---|---|---|---|---|---|
| Prescription | Prescription 1 | Prescription 2 | Prescription 3 | Prescription 4 | API Control |
| Instrument | Buchi Nanometer Spray Drying Equipment B-90 HP | | | | |
| Pattern | Inert Gas, Closed Loop | | | | |
| Drying Airflow | Nitrogen (containing a little carbon dioxide) | | | | |
| Content of Oxygen | < 4% | | | | |
| Size of Atomizer | Medium | | | | |
| Frequency | 120 kHz | | | | |
| Rate of Sampling Pump | 70% | | | | |
| Spray Rate | 70% | | | | |
| Operating Frequency of Air Sniffer | 30 Hz | | | | |
| Dry Gas Flow | 120 L/min | | | | |
| Relative Pressure of Drying Tube | 40 hPa | | | | |
| Inlet Temperature | 80°C | | | | |
| Outlet Temperature | 40°C - 45°C | | | | |
| Yield | 79% | 63% | 73% | 70% | 57% |

### Physical characterization on amorphous solid dispersion powder obtained through preparation

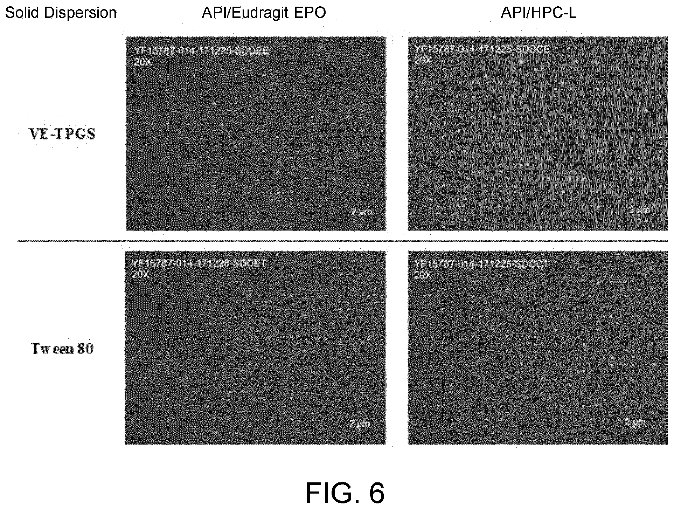

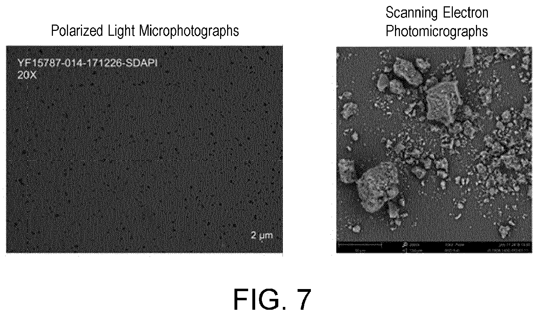

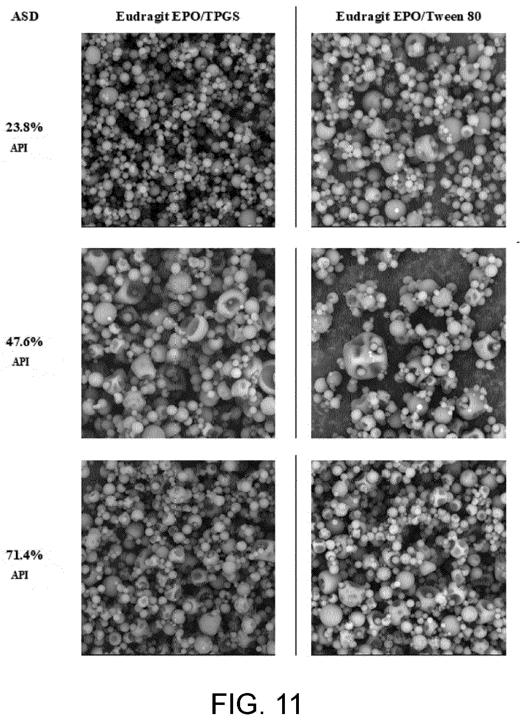

The physical characterization results of the prepared spray drying solid dispersions are shown in Table 17, and the specific characterization reports are shown in Fig. 6 to Fig. 8. The results of a polarized light microphotograph (as shown in Fig. 6) and an X-ray diffraction pattern (as shown in Fig. 8) show that all the prepared spray drying dispersions are amorphous. In addition, in the polarized light microphotograph (as shown in Fig. 7), trace crystals are observed in the background for unknown reasons. The scanning electron microscope results of the Eudragit EPO system are shown in Fig. 11, wherein the solid dispersions with TPGS as the functional excipient are mostly sphere, with the diameter of about 0.5-3 µm, and the solid dispersions with Tween-80 as the functional excipient are mostly depressed sphere, with the diameter of about 0.5-4 µm. In addition, a physical topography of the untreated rifamycin - nitroimidazole coupling molecule API is shown in Fig. 7 as reference.

**Table 17 Characterization on Spray Drying Dispersions of Rifamycin - Nitroimidazole Coupling Molecule/Excipient/Surfactants**

| Item | Result | | | | |
|---|---|---|---|---|---|
| Prescription | Prescription 1 | Prescription 2 | Prescription 3 | Prescription 4 | API Control |
| Component | API (23.8%) /EPO/ VE-TPGS | API (23.8%) /HPC-L/ VE-TPGS | API (23.8%) /EPO/ Tween 80 | API (23.8%) /HPC-L/ Tween 80 | Rifamycin - Nitroimidazole Coupling Molecule |
| Physical Appearance | Purple Powder | Purple Powder | Purple Powder | Purple Powder | Purple Powder |
| Drug Loadings (w%, HPLC) | 26.5 | 25.2 | 27.0 | 26.1 | 110% |
| Total Amount of Related Substance (w%, HPLC) | 0.5 | 0.2 | 0.2 | 0.2 | 0.4 |
| Microstructure (XRD) | Amorphous | Amorphous | Amorphous | Amorphous | Amorphous |
| Microstructure (PLM) | Amorphous | Amorphous | Amorphous | Amorphous | Amorphous |
| Particle Diameter (SEM) | <5 µm | <5 µm | <5 µm | <5 µm | <5 µm |
| Glass Transition Temperature (°C, MDSC) | 57.0, 136.8 | N/A | 58.1 | N/A | 145.0 |

Dissolution experiment on amorphous solid dispersion powder obtained through preparation

About 344 mg of prepared solid dispersions are taken and poured into a 40 mL glass bottle; 20 mL of acetate buffer of pH 4.5 (preheated to 37°C) is used as the dissolution medium (the target concentration of the rifaomycin - nitroimidazole coupling molecule is 4 mg/mL); the glass bottle is sealed; and the above materials are whirled for 5 seconds and placed on a heated magnetic stirrer at 37°C after a stir bar is inserted. The rotational speed is 300 rpm. After 10 min, 20 min, 40 min, 60 min, 90 min, 120 min and 180 min, 500µL of the liquid is taken into the centrifuge tube and centrifuged at 14000 rpm for 3 min. The supernatant liquid is taken into the liquid phase bottle and diluted to 10 times with acetonitrile as the solution to be tested, the high performance liquid chromatography is injected and the chromatogram is recorded, and the concentration is calculated by the peak area using the external standard method.

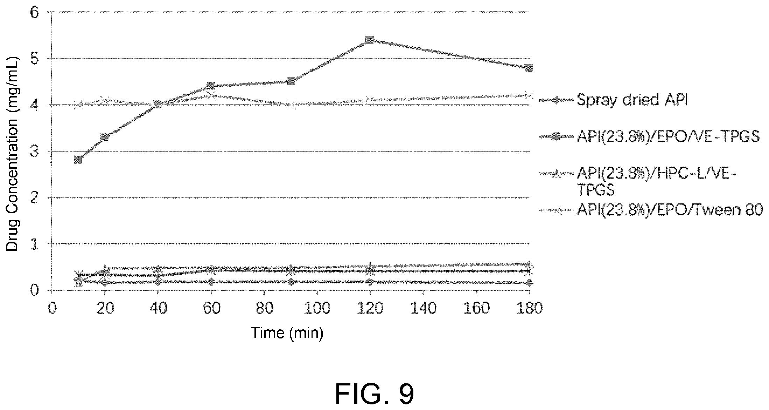

The dissolution results of the spray drying solid dispersions are shown in Table 18 and Fig. 9. In the acetate buffer of pH 4.5, Eudragit ^{®} EPO and TPGS or Tween-80, as the solid dispersions of the excipient, can obviously improve the dissolution rate and degree of the rifamycin - nitroimidazole coupling molecule. On the contrary, the spray drying rifamycin - nitroimidazole coupling molecule/HPC-L/surfactant has no obvious improvement effect on the dissolution of the rifamycin - nitroimidazole coupling molecule. In contrast to VE-TPGS and Tween-80 as functional excipient, Tween 80 can more obviously make the rifamycin - nitroimidazole coupling molecule rapidly release to 4 mg/mL in 10 min, while it takes 40 min for VE-TPGS to achieve similar release effect, and the concentration is only 2.8 mg/mL in 10 min. Therefore, Tween80 is considered to have more strong rapid release effect. In addition, the two-hour solubility of the spray drying rifamycin - nitroimidazole coupling molecule is similar to the two-hour solubility of rifamycin - nitroimidazole coupling molecule API (0.15mg/mL, Table 14), showing that the spray drying API with smaller particle size has no obvious effect on equilibrium solubility.

**Table 18 Dissolution Results of Solid Dispersion Powder in Acetate Buffer of pH 4.5 (37°C)**

| Spray Drying System | Drug Concentration (mg/mL) | | | | | | | pH | Phenomenon |
|---|---|---|---|---|---|---|---|---|---|
| | 10 Min | 20 Min | 40 Min | 60 Min | 90 Min | 120 Min | 180 Min | | |
| Spray Drying Rifamycin - Nitroimidazole Coupling Molecule | 0.21 | 0.17 | 0.19 | 0.18 | 0.18 | 0.18 | 0.16 | 4.47 | Inhomogeneous Suspension |
| Prescription 1 | 2.8 | 3.3 | 4.0 | 4.4 | 4.5 | 5.4 | 4.8 | 5.73 | Inhomogeneous Suspension |
| Prescription 2 | 0.16 | 0.47 | 0.48 | 0.49 | 0.48 | 0.52 | 0.57 | 4.51 | Inhomogeneous Suspension |
| Prescription 3 | 4.0 | 4.1 | 4.0 | 4.2 | 4.0 | 4.1 | 4.2 | 5.56 | Inhomogeneous Suspension |
| Prescription 4 | 0.34 | 0.34 | 0.32 | 0.43 | 0.41 | 0.41 | 0.42 | 4.53 | Inhomogeneous Suspension |

In this embodiment, the prescription drug loading of solid dispersion is screened.

According to the results of the dissolution experiment of the spray-dried dispersions, Eudragit ^{®} EPO is selected as the carrier for the solid dispersion formulation. In order to further investigate the effect of drug loading on the spray-dried dispersions, the solid dispersions that contain rifamycin-nitroimidazole coupling molecule and carrier in a ratio of 1:1 and 1:3 and also contain VE-TPGS or Tween 80 5%(w/w) are prepared, and the components of the solid dispersions are shown in Table 19. Acetone is used as the spray drying solvent and the process parameters are listed in Table 20. The solid dispersions prepared by spray drying are dried in a 35°C vacuum drying oven for 40 hours and stored in a -20°C refrigerator.

**Table 19 Composition of Solid Dispersions with Different Drug Loadings**

| No. | Component | | |
|---|---|---|---|
| | API + Subsidiary Materials + Surfactant | Ratio | Mass (mg) |
| Prescription 5 | API + Eudragit EPO + TPGS | 10:10:1 | 500 + 500 + 50 |
| Prescription 6 | API + Eudragit EPO + Tween80 | 10:10:1 | 500 + 500 + 50 |
| Prescription 7 | API + Eudragit EPO + TPGS | 15:5:1 | 600 + 200 + 40 |
| Prescription 8 | API + Eudragit EPO + Tween80 | 15:5:1 | 600 + 200 + 40 |

**Table 20 Spray Drying Process Parameters**

| Item | Parameter | | | |
|---|---|---|---|---|
| Prescription | Prescription 5 | Prescription 6 | Prescription 7 | Prescription 8 |
| Instrument | Buchi Nanometer Spray Drying Equipment B-90 HP | | | |
| Pattern | Inert Gas, Closed Loop | | | |
| Drying Airflow | Nitrogen (containing a little carbon dioxide) | | | |
| Content of Oxygen | < 4% | | | |
| Size of Atomizer | Large | | Medium | |
| Frequency | 120 kHz | | | |
| Rate of Sampling Pump | 70% | | | |
| Spray Rate | 70% | | | |
| Operating Frequency of Air Sniffer | 30 Hz | | | |
| Dry Gas Flow | 115 L/min | | | |
| Relative Pressure of Drying Tube | 40 hPa | | | |
| Inlet Temperature | 80°C | | | |
| Outlet Temperature | 40-45°C | | | |
| Yield | 73% | 72% | 68% | 72% |

The solid dispersions obtained according to prescriptions 5-8 are characterized.

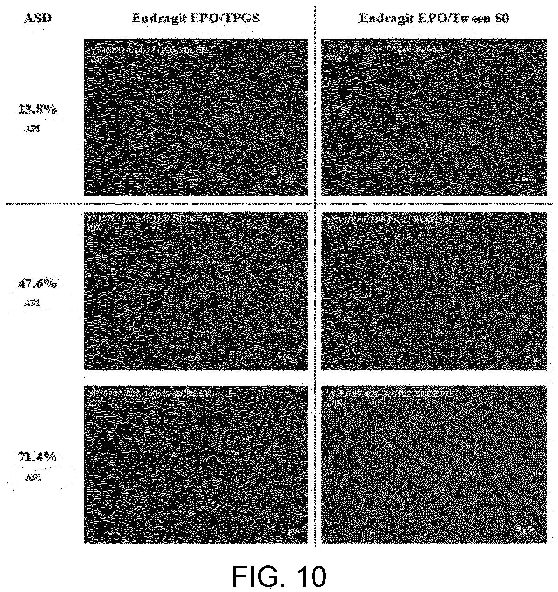

The physical characterization results of the spray-dried solid dispersions with different drug loadings are shown in Table 21, and specific characterizations are shown in Figs. 10-19. Meanwhile, the content of drugs and related substances in the spray-dried dispersions is analyzed by high-performance liquid chromatography (HPLC).

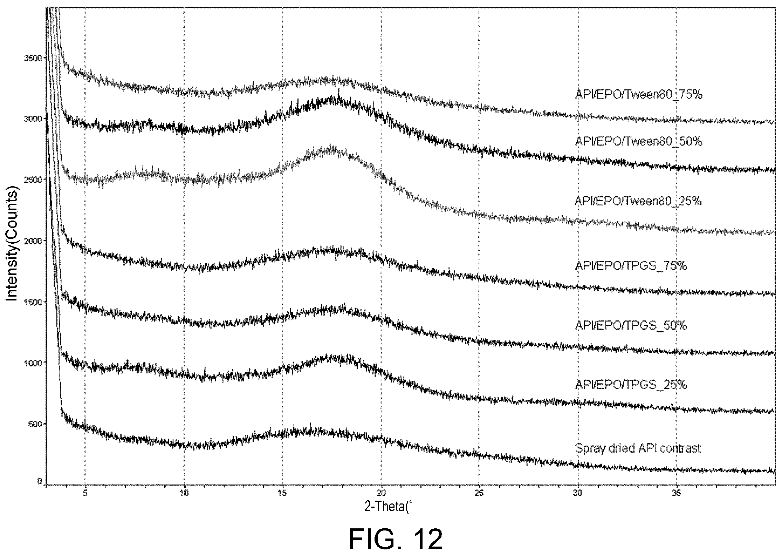

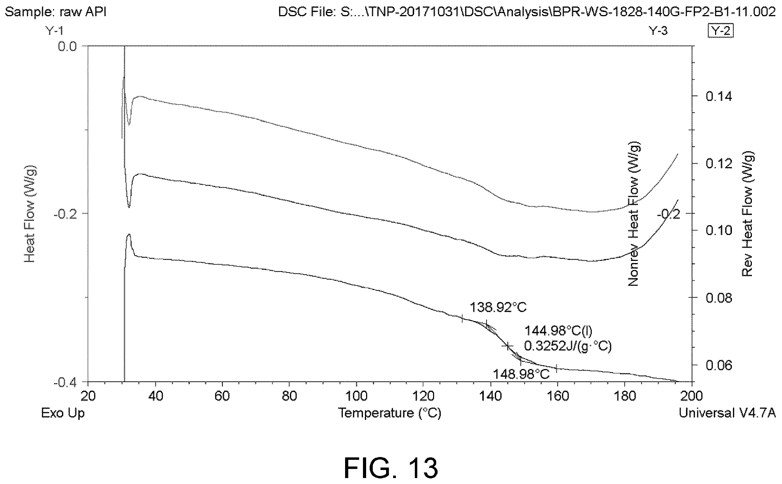

Polarized light microphotographs (as shown in Fig. 10) and X-ray diffraction diagram (as shown in Fig. 12) show that all the prepared spray-dried dispersions with different drug loadings are amorphous. The scanning electron photomicrographs (as shown in Fig. 11) show that when the drug loading is higher than 50%, the degree of indentation of the spray-dried particle surface will be increased, which is presumably caused by the different diffusion rates of rifamycin-nitroimidazole coupling molecule and Eudragit ^{®} EPO during the drying process. MDSC results show that the glass transition temperature Tg of active ingredient rifamycin-nitroimidazole coupling molecule is about 145.0°C (Fig. 13). All the spray-dried dispersions with different drug loadings are single-phase solid dispersions (as shown in Figs. 14-19), and generally conform to the Gordon-Taylor law, except that API (23.8%)/EPO/TPGS spray-dried dispersions have two glass transition temperatures, indicating that the system is non-single phase. In addition, Tg of the solid dispersion in Tween-80 system is generally lower than that in VE-TPGS system. In the HPLC content analysis (Table 21), the actual drug content of the solid dispersions is slightly higher than the theoretical drug content. In addition, the content of related substances does not increase during the spray drying process.

**Table 21 Physical Characterization Results of Solid Dispersion Powder with Different Drug Loadings Capacity**

| Item | Result | | | |
|---|---|---|---|---|
| Prescription | Prescription 5 | Prescription 6 | Prescription 7 | Prescription 8 |
| Physical Appearance | Purple Powder | Purple Powder | Purple Powder | Purple Powder |
| Content (w%, HPLC) | 53.6 | 54.2 | 81.5 | 81.8 |
| Related Substance (w%, HPLC) | 0.0 | 0.0 | 0.33 | 0.33 |
| Microstructure (XRD) | Amorphous | Amorphous | Amorphous | Amorphous |
| Microstructure (PLM) | Amorphous | Amorphous | Amorphous | Amorphous |
| Particle Diameter (SEM) | 0.5 - 5 µm | 0.5 - 5 µm | 0.5 - 5 µm | 0.5 - 5 µm |
| Surface Topography (SEM) | Depressed Sphere | Depressed Sphere | Depressed Sphere | Depressed Sphere |
| Tg (°C, MDSC) | 75.0 | 69.9 | 94.2 | 91.5 |

The powder dissolution experiments are carried out on the obtained solid dispersions.

A proper number of the solid dispersions are respectively poured into 40 mL glass bottles; 20 mL of pH 4.5 acetate buffer (preheated to 37°C) is used as the dissolution medium (the target concentration of the rifaomycin-nitroimidazole coupling molecule is 10 mg/mL); the glass bottles are sealed; and the above materials are whirled for 5 seconds and then placed on a heated magnetic stirrer at 37°C after a stir bar is inserted. The rotational speed is 300 rpm. After 10 min, 20 min, 40 min, 60 min, 90 min, 120 min, and 180 min, 500µL of the liquid is transferred into a centrifuge tube and centrifuged at 14000 rpm for 3 min. The supernatant liquid is taken into a liquid phase bottle, and diluted to one tenth with acetonitrile as the solution to be tested. The solution to be tested is injected into a high performance liquid chromatography to record chromatograms. The concentration is calculated by the peak area using the external standard method.

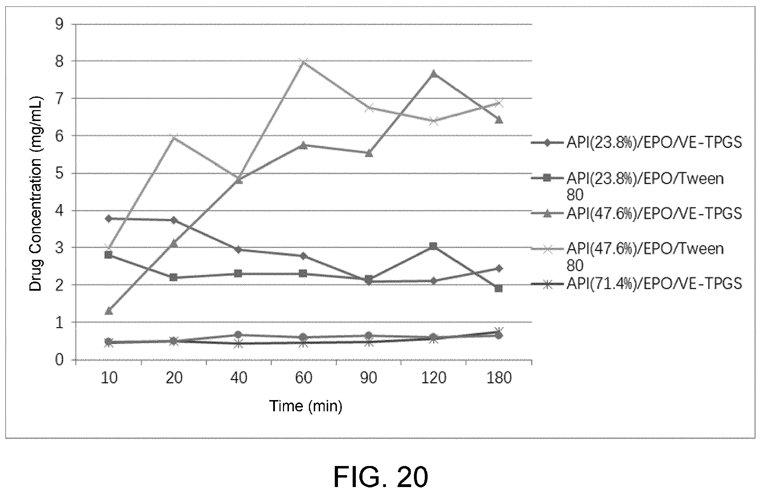

Dissolution results of the spray-dried solid dispersions with different drug loadings are shown in Table 22 and Fig. 20. The dissolution test results show that the spray-dried dispersions of the rifamycin-nitroimidazole coupling molecule/Eudragit ^{®} EPO/Tween-80 (47.6%/47.6%/4.8%) have the best dissolution behavior, and the dissolution rate reaches 3 mg/mL after 10 minutes and 7 mg/mL after 3 hours. The spray-dried dispersions of the rifamycin-nitroimidazole coupling molecule/Eudragit ^{®} EPO/Tween-80 (23.8%/71.4%/4.8%) have the best rapid release effect of all the prescriptions. But as the Eudragit ^{®} EPO is continuously dissolved out, the pH of the solution system increases continuously, which inhibits the solubility of the rifamycin-nitroimidazole conjugate molecule, so that the rifamycin-nitroimidazole conjugate molecule cannot be maintained at a stable and high-level concentration. However, the dissolution behavior of the system with a drug loading of 71.4% is poor, which may result from low subsidiary materials content, poor wettability and poor capability to maintain supersaturation. After a comprehensive consideration, the drug loading of 47.6% is selected as the optimal drug ratio of prescription.

**Table 22 Dissolution Results of Solid Dispersion Powder in pH 4.5 Acetate Buffer (37 °C)**

| Solid | Drug Concentration (mg/mL) | | | | | | | Final | Physical |
|---|---|---|---|---|---|---|---|---|---|
| Dispersion | 10 min | 20 min | 40 min | 60 min | 90 min | 120 min | 180 min | pH | Appearance (3 hours) |
| Prescription 1 | 3.8 | 3.7 | 3.0 | 2.8 | 2.1 | 2.1 | 2.4 | 6.52 | Suspension |
| Prescription 3 | 2.8 | 2.2 | 2.3 | 2.3 | 2.1 | 3.0 | 1.9 | 6.33 | |
| Prescription 5 | 1.3 | 3.1 | 4.8 | 5.8 | 5.5 | 7.7 | 6.4 | 5.34 | |
| Prescription 6 | 3.0 | 6.0 | 4.9 | 8.0 | 6.8 | 6.4 | 6.9 | 5.37 | |
| Prescription 7 | 0.47 | 0.50 | 0.44 | 0.46 | 0.48 | 0.56 | 0.76 | 4.66 | |
| Prescription 8 | 0.48 | 0.49 | 0.67 | 0.61 | 0.65 | 0.61 | 0.65 | 4.68 | |

The study on the short-term physical stability of the solid dispersions obtained according to prescriptions 1, 3, 5-8 is performed.

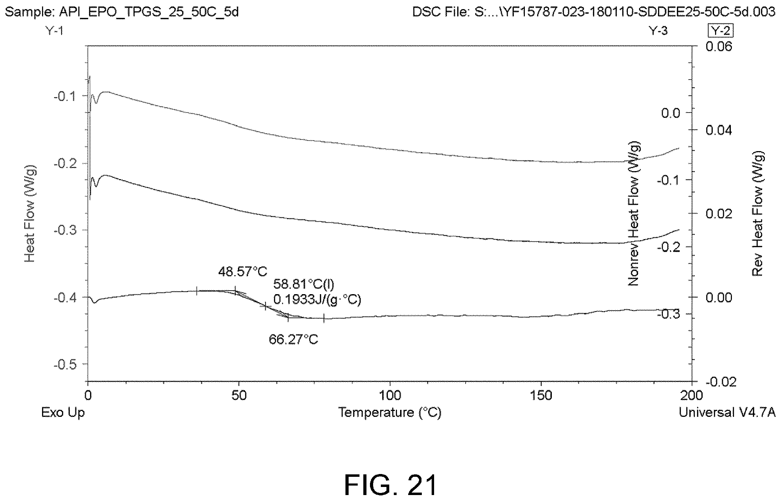

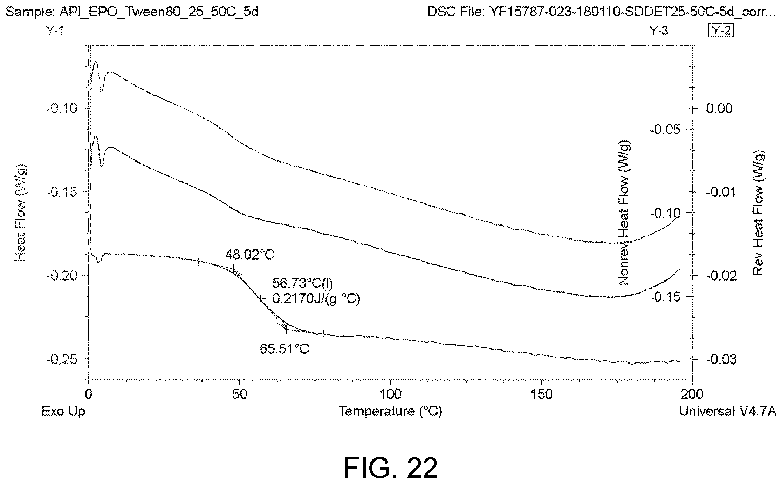

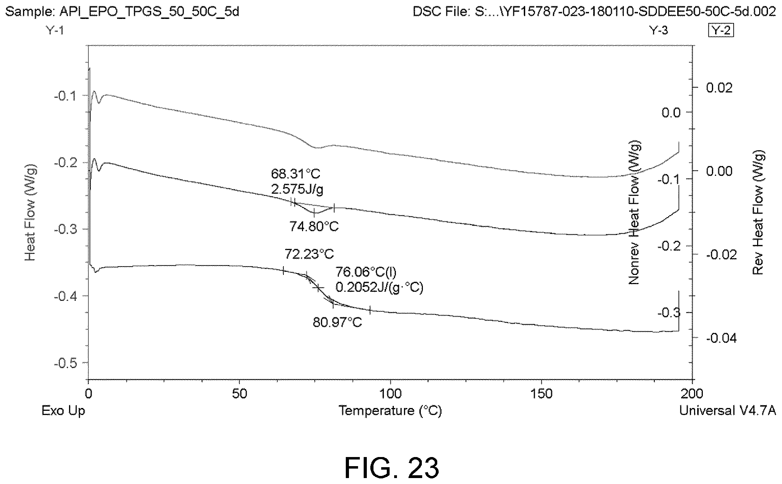

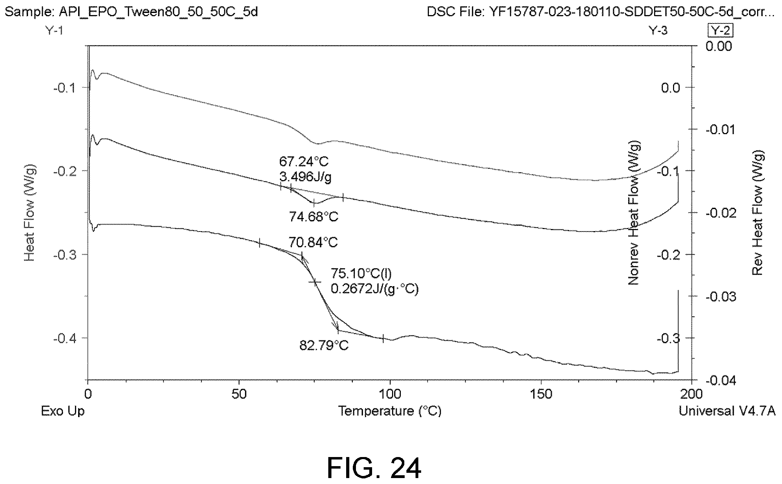

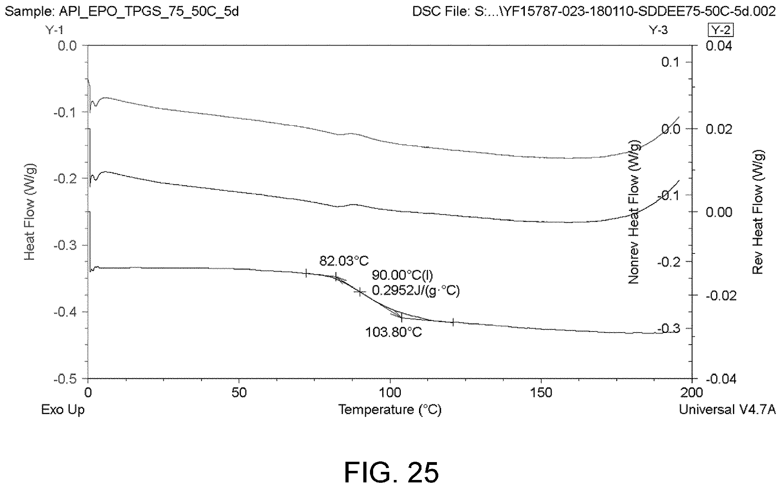

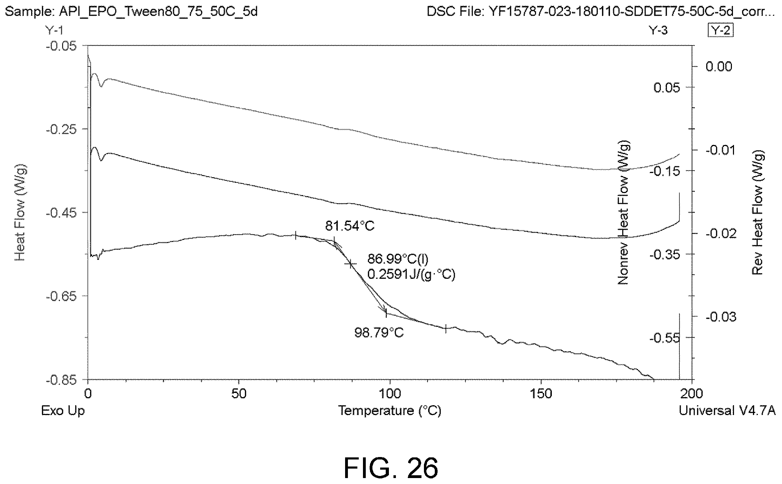

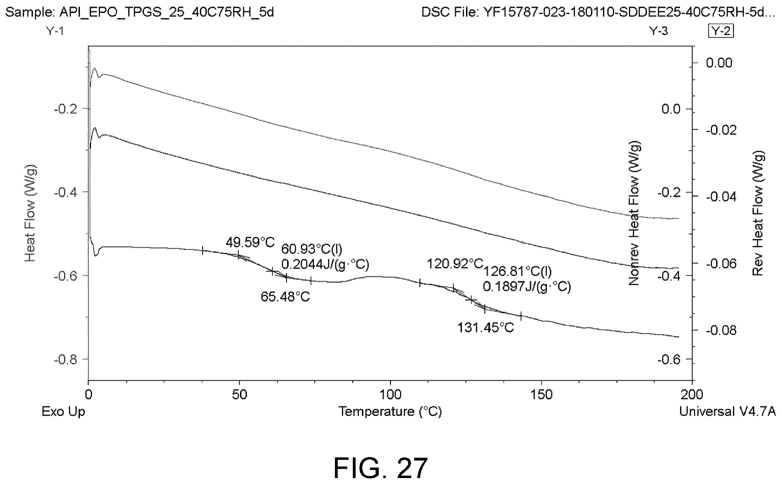

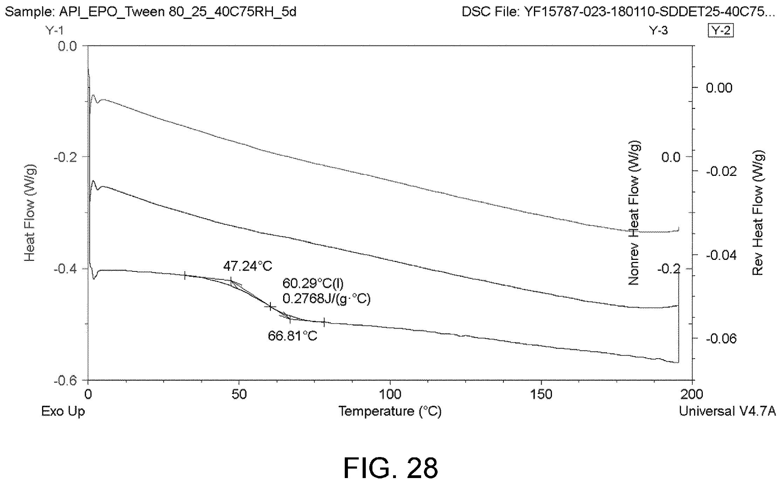

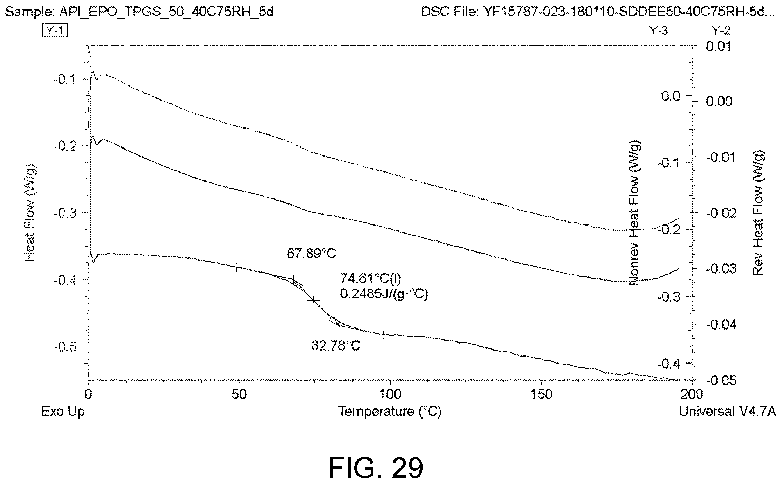

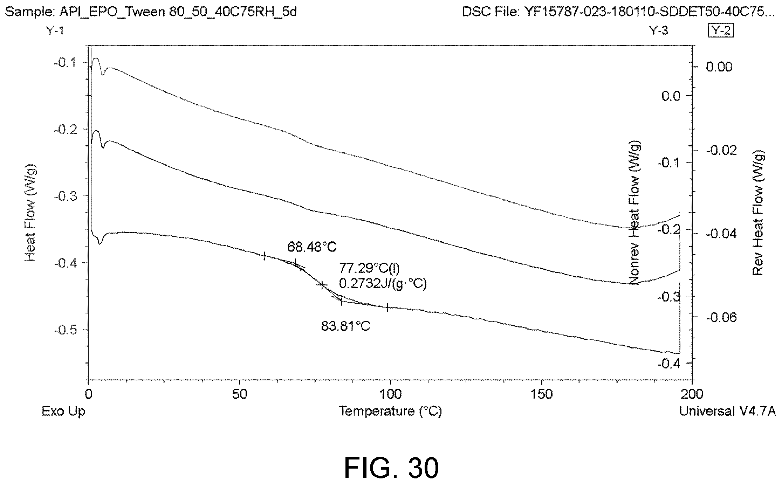

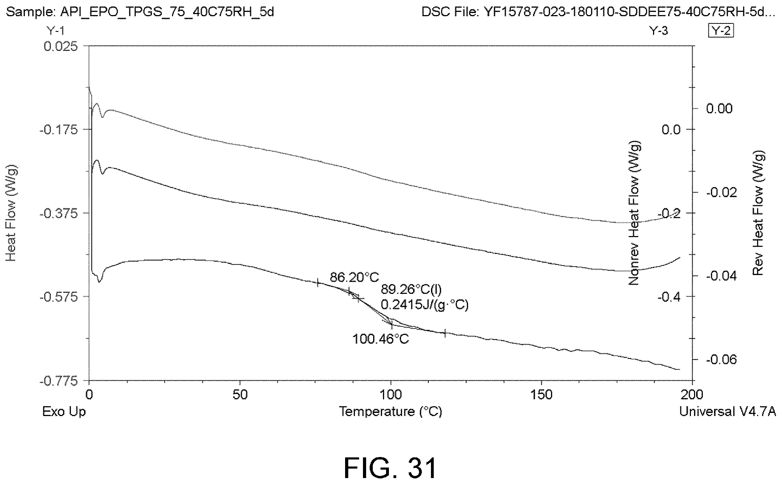

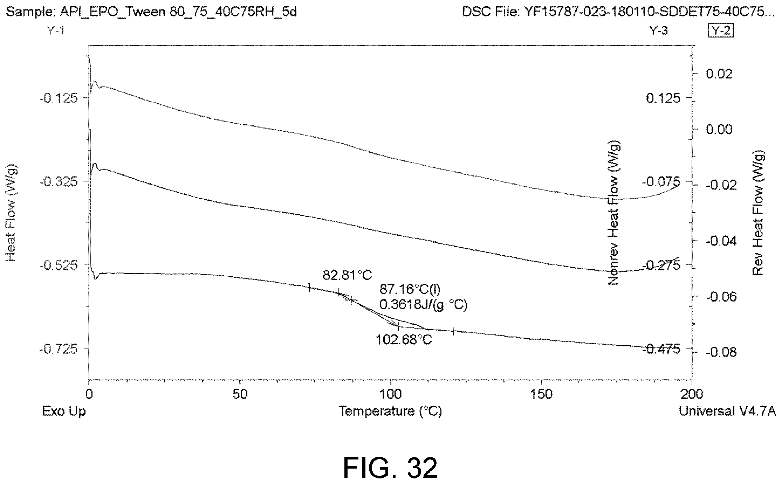

An appropriate number of the spray-dried dispersions of the rifamycin-nitroimidazole coupling molecule/EPO/TPGS (or Tween-80) are weighed and put into a 1.5 mL liquid phase bottle, and then placed into a 50°C and 40°C /75% RH stability chamber. The porous aluminum-foil paper is used to protect against light and prevent cross contamination. After five days, MDSC characterization is performed, and the results are shown in Table 23 and Figs. 21-32.

At 50°C and 40°C/75% RH, the Tg value of amorphous solid dispersion (ASD) powder with different drug loadings varies, but there is still only one Tg, that is, a single solid-solution phase, and there is no obvious exothermic signal associated to a crystallization process, indicating that the solid dispersion system has good physical stability under short-term acceleration condition.

**Table 23 Changes in Glass Transition Temperature of Solid Dispersion Powder during Physical Stability Experiments (Conditions: 50 °C and 40 °C/75% RH)**

| Prescription | | Prescription 1 | Prescription 3 | Prescription 5 | Prescription 6 | Prescription 7 | Prescription 8 |
|---|---|---|---|---|---|---|---|
| Component | | API (23.8%) /EPO/ VE-TPGS | API (23.8%) /EPO/ Tween-80 | API (47.6%) /EPO/ VE-TPGS | API (47.6%) /EPO/ Tween-80 | API (71.4%) /EPO/ VE-TPGS | API (71.4%) /EPO/ Tween-80 |
| Initial Condition | Tg, °C | 57.0, 136.8 | 58.1 | 75.0 | 59.9 | 94.2 | 91.5 |
| 50 °C, 5 Days | Tg, °C | 58.8 | 56.7 | 76.1 | 75.1 | 90.0 | 87.0 |
| 40 °C /75% RH, 5 Days | Tg, °C | 50.9, 126.8 | 60.3 | 74.6 | 77.3 | 89.3 | 87.2 |

The study on the long-term physical stability of the solid dispersions obtained according to prescription 3 is performed.

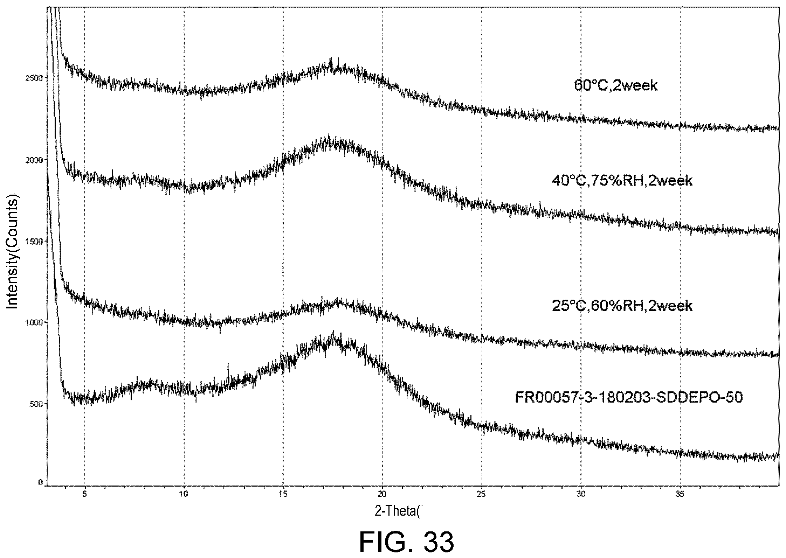

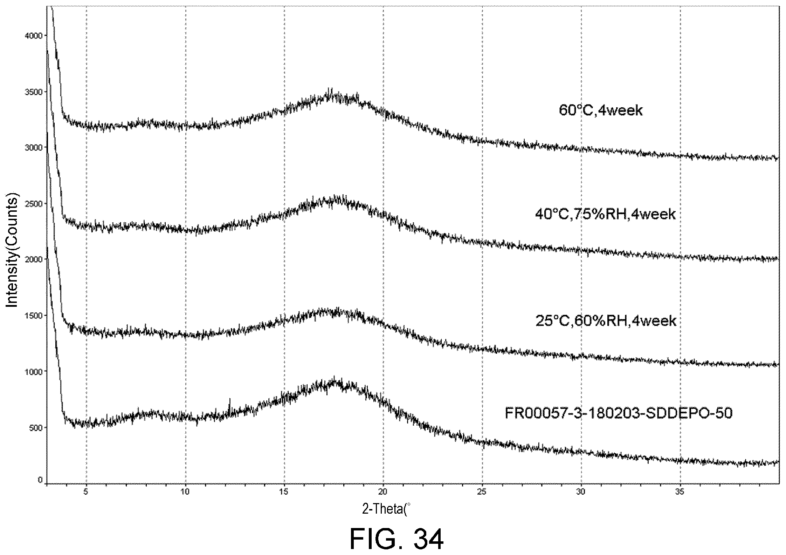

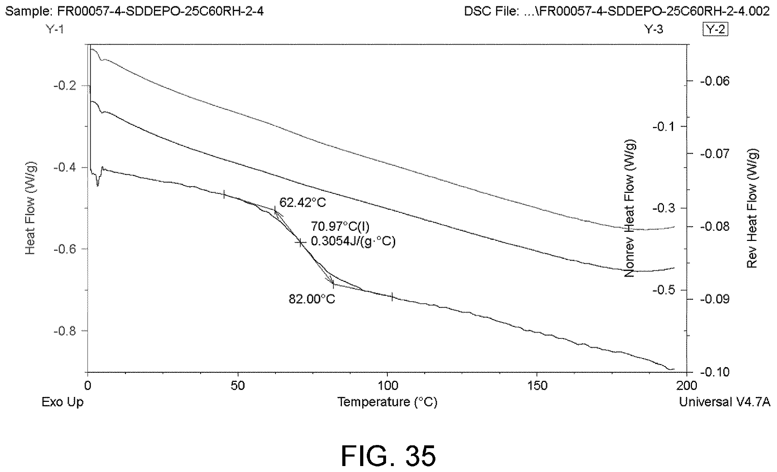

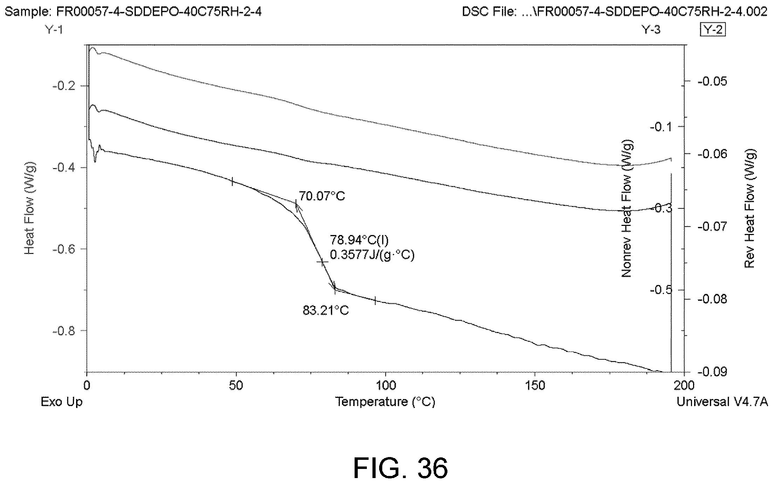

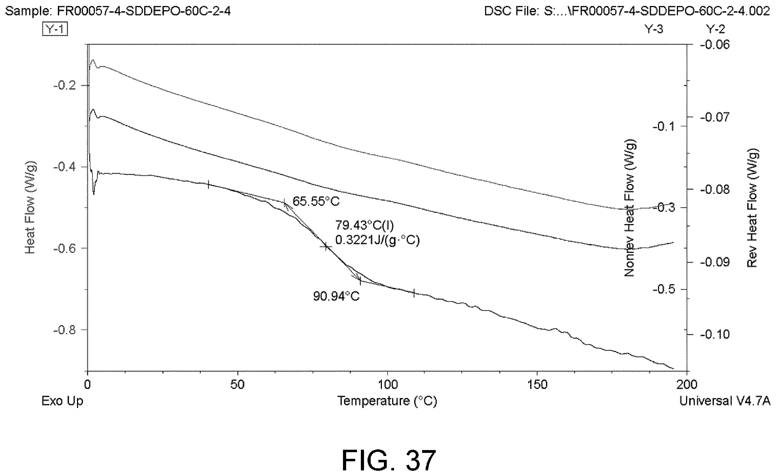

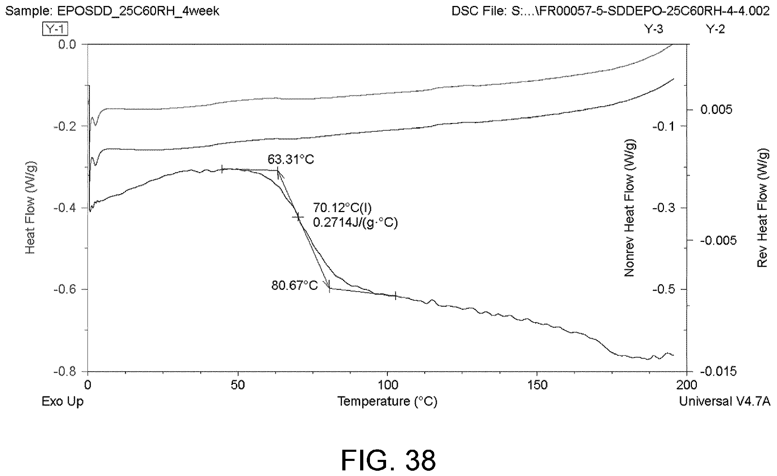

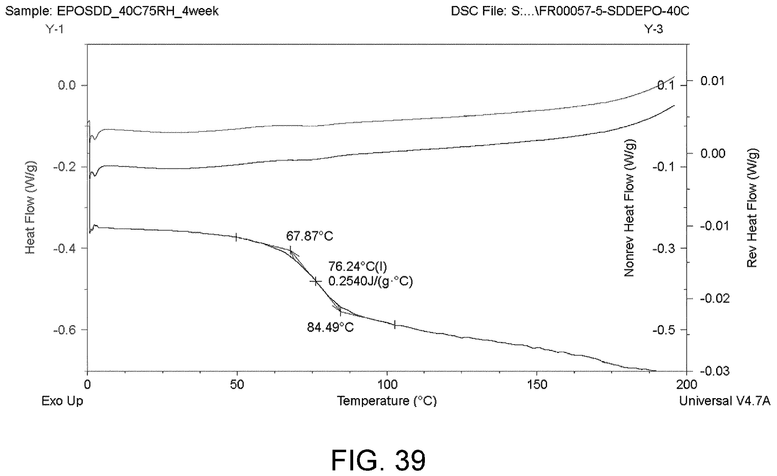

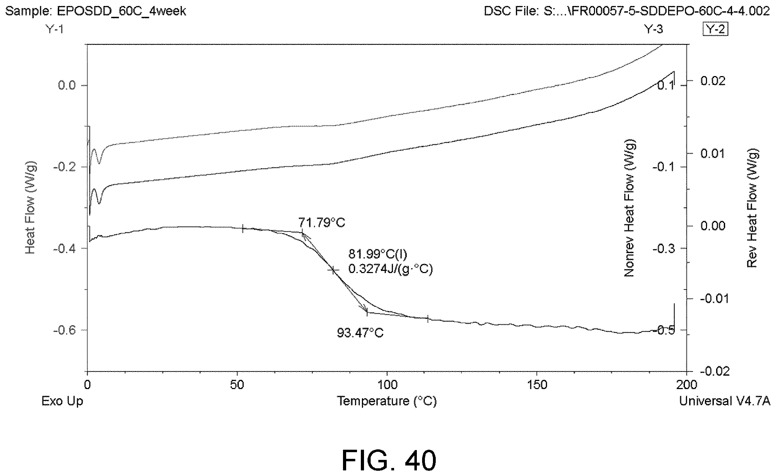

An appropriate number of the spray-dried dispersions of the rifamycin-nitroimidazole coupling molecule/EPO/Tween-80 are weighed and put into a 40 mL glass bottle, and then respectively placed into 25 °C/60% RH, 40°C/75% RH and 60 °C stability chambers. The porous aluminum-foil paper is used to protect against light and prevent cross contamination. XRPD and MDSC characterizations are respectively performed after 2 weeks and 4 weeks, and the results are shown in Figs. 33-40.

X-ray diffraction diagrams show that the solid dispersions are still the same and uniformly distributed amorphous system after 4 weeks. MDSC shows that the glass transition temperature (Tg) decreases slightly. According to the experimental results, ASD powder of the rifaomycin-nitroimidazole coupling molecule/Eudragit ^{®} EPO/Tween 80 (47.6%/47.6%/4.8%) has good physical stability.

In this embodiment, the contrast experiment of the solid dispersion formulation of rifamycin-nitroimidazole coupling molecule and other conventional formulations (for example, capsules) is also provided.

The common capsules of the rifamycin-nitroimidazole coupling molecule are the capsules with specification of 100 mg (batch number: D-1709FP2300-05-HPMC and D-1709FP2300-05-HG) which are obtained by filling the solid particles prepared by dry granulation process into the corresponding capsule shells. The granulation process comprises mixing, dry granulation and lubrication. The subsidiary materials used are common commercial pharmaceutic subsidiary materials, including filler/diluent, disintegrant, glidant, surfactant and lubricant. The capsule shells used are hydroxypropyl methylcellulose capsule shells (HPMC) and gelatin capsule shells (HG). The solid dispersion capsules of the rifamycin-nitroimidazole coupling molecule are the capsules with specification of 100 mg (batch number: FR00057-3-180203-SDDEPO-50-HPMC and FR00057-3-180203-SDDEPO-50-HG) which are obtained by directly filling the above preferable scale-up batches of spray-dried solid dispersions (batch number: FR00057-3-180203-SDDEPO-50) into the corresponding capsule shells.

Dissolution experiments on the capsules (specification of 100 mg) of the rifamycin-nitroimidazole coupling molecule are performed.

A dissolution tester (paddle method) in accordance with the requirements of *Chinese Pharmacopoea* is used, pH 4.5 acetate buffer (preheated to 37°C) is selected as the dissolution medium, and the rotational speed is 75 rpm. The above materials are sampled respectively after 10 min, 15 min, 20 min, 30 min, 45 min and 60 min, and are sampled again after the tester is operated at 250 rpm for 30 min. 5 mL of liquid is taken and filtered by a 0.45µm PTFE filter. The obtained liquid is injected into the high performance liquid chromatography to record chromatograms. The concentration is calculated by the peak area using the external standard method.

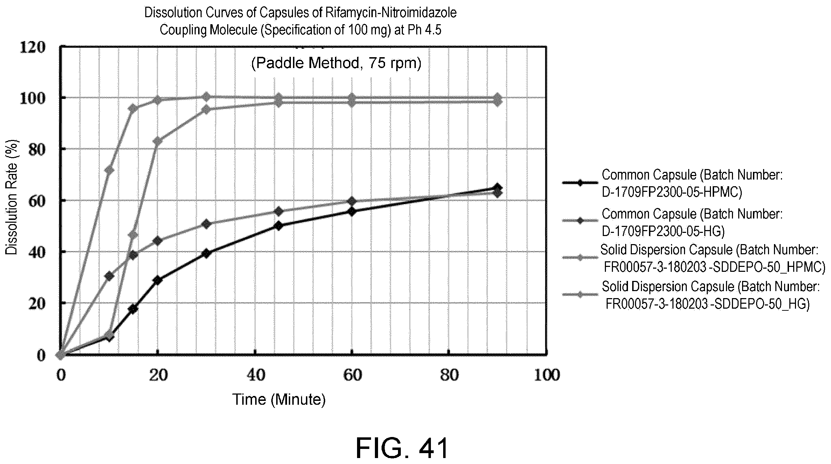

The dissolution results of the solid dispersion capsules of rifamycin-nitroimidazole coupling molecule and common dry granulation capsules are shown in Table 24 and Fig. 41. The dissolution test results show that the solid dispersion capsules of the rifamycin-nitroimidazole coupling molecule have the best dissolution behavior. The dissolution rate of the solid dispersion capsules (batch number: FR00057-3-180203-SDDEPO-50-HG) reaches 72% after 10 minutes and 96% after 15 minutes. The solid dispersion capsule is obviously better than common dry granulation capsule (batch number is D-1709FP2300-05-HG, and the dissolution rate reaches 31% after 10 minutes and 39% after 15 minutes).

**Table 24 Dissolution Rate of Capsules of Rifamycin-Nitroimidazole Coupling Molecule (Specification of 100 mg) in pH 4.5 Acetate Buffer (37 °C)**

| Capsule (Batch Number) | | Time (min)/Average Dissolution Rate (%) | | | | | | |
|---|---|---|---|---|---|---|---|---|
| | | 10 | 15 | 20 | 30 | 45 | 60 | 90 |
| Solid Dispersion Capsule | FR00057-3-180203-SDDEPO-50-HG | 72 | 96 | 99 | 100 | 100 | 100 | 100 |
| | FR00057-3-180203-SDDEPO-50-HPMC | 8 | 47 | 83 | 96 | 98 | 98 | 98 |
| Common Capsule | D-1709FP2300-05-HG | 31 | 39 | 44 | 51 | 56 | 60 | 63 |
| | D-1709FP2300-05-HPMC | 7 | 18 | 29 | 39 | 50 | 56 | 65 |

The specific embodiment of this description also provides a granule which comprises the above solid dispersion of the rifamycin-nitroimidazole coupling molecule. The granule may either be a conventional granule or a in-granule controlled release formulation, which is obtained by making the solid dispersions of rifamycin-nitroimidazole coupling molecule into a granule. The granule is disintegrated by granule to release active components (rifamycin-nitroimidazole coupling molecule).

The specific embodiment of this description also provides a pharmaceutical composition which comprises the above granule.

The specific embodiment of this description also provides a pharmaceutical composition which comprises the above solid dispersion of the rifamycin-nitroimidazole coupling molecule. The composition is a composition containing the solid dispersions of the rifamycin-nitroimidazole coupling molecule and other subsidiary materials, and may also include a pharmacologically acceptable carrier. Further, the subsidiary materials include one or more of excipients, such as diluent, adhesive, lubricant, in-granule controlled release formulation, disintegrant, colorants, corrigent or sweetener. The above composition may be formulated for the preparation of coated and uncoated tablets, hard and soft gelatin capsules, sugar-coated pill, lozenge, dry film, pellet, and powder in sealed packages. The above composition may be formulated for the preparation of the locally used formulations such as ointment, pomade, cream, gelata and lotion. The "pharmaceutically acceptable carrier" includes pharmaceutically acceptable material, composition or medium, such as liquid or solid filler, diluent, excipient, solvent or coating material, which assist in carrying or transporting the chemicals in the present invention from one organ or part of the organism to another organ or part of the organism. Each carrier is preferably "acceptable" in the sense that the carrier is suitable for other components of the formulation and is harmless to the subject. Some examples of the materials that may be used as the pharmaceutically acceptable carrier include: (1) sugar, such as lactose, dextrose and sucrose; (2) starch, such as cornstarch and potato starch; (3) cellulose and derivatives thereof, such as sodium carboxymethyl cellulose, ethyl cellulose and cellulose acetate; (4) powdery gum tragacanth; (5) malt; (6) gelatin; (7) talcum powder; (8) excipient, such as cocoa butter and suppository wax; (9) oil, such as peanut oil, cottonseed oil, safflower oil, sesame oil, olive oil, corn oil and soybean oil; (10) diols, such as propylene glycol; (11) polyol, such as glycerine, sorbitol, mannitol and polyethylene glycol; (12) ester, such as ethyl oleate and ethyl laurate; (13) agar; (14) buffer, such as magnesium hydroxide and aluminum hydroxide; (15) alginic acid; (16) pyrogen-free water; (17) isotonic saline solution; (18) Riger's solution; (19) ethanol; (20) phosphate buffered solution; and (21) other non-toxicity suitable substances.

The specific embodiment of this description also provides an application of the above granule in preparation of a drug for treating diseases caused by microbial infection.

The specific embodiment of this description also provides an application of the above pharmaceutical composition in preparation of a drug for treating diseases caused by microbial infection.

The specific embodiment of this description also provides an application of the above solid dispersion of the rifamycin-nitroimidazole coupling molecule or the composition thereof in preparation of a drug for treating diseases caused by microbial infection.

## Claims

1. A solid dispersion of a rifamycin-nitroimidazole coupling molecule, comprising a rifamycin-nitroimidazole coupling molecule of a structure shown in formula I, a polymer carrier, a functional excipient and a solvent,
wherein the polymer carrier comprises one or a combination of more of HPC-L, HPMC E3, Soluplus ^{®} and polymethacrylate;
the functional excipient comprises one or a combination of more of sodium lauryl sulfate, meglumine, VE-TPGS and Tween 80;

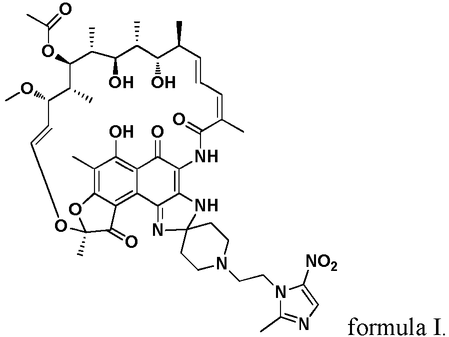

2. The solid dispersion according to claim 1, wherein the polymethacrylate comprises Eudragit ^{®} EPO.

3. The solid dispersion according to claim 1, **characterized in that**, the solid dispersion is **characterized by** one or more XRPDs basically similar to Fig. 8.

4. The solid dispersion according to claim 1, **characterized in that**, the solid dispersion is **characterized by** one or more thermograms basically similar to Fig. 14-Fig. 19.

5. The solid dispersion according to claim 1, **characterized in that**, based on the total mass of the rifamycin-nitroimidazole coupling molecule, the polymer carrier and the functional excipient as 100%, the use amount of the rifamycin-nitroimidazole coupling molecule is 23.8%-71.2%, the use amount of the polymer carrier is 23.8%-71.2%, and the use amount of the functional excipient is 3%-7%;
preferably, the polymer carrier comprises HPC-L and/or Eudragit ^{®} EPO;
preferably, the functional excipient are VE-TPGS and/or Tween 80.

6. The solid dispersion according to claim 1, **characterized in that**, the solvent comprises one or a combination of more of methanol, ethanol, acetone, ethyl acetate, tetrahydrofuran and dichloromethane.

7. The solid dispersion according to claim 1, **characterized in that**, when the solid dispersion is placed at 40°C/75%RH or 60°C/open conditions for 4 weeks, the solid dispersion remains amorphous according to XRPD.

8. A granule, comprising the solid dispersion of the rifamycin-nitroimidazole coupling molecule of any one of claims 1-7.

9. A pharmaceutical composition, comprising the granule of claim 8.

10. A pharmaceutical composition, comprising the solid dispersion of the rifamycin-nitroimidazole coupling molecule of any one of claims 1-7.

11. An application of the solid dispersion of the rifamycin-nitroimidazole coupling molecule of any one of claims 1-7 in preparation of a drug for treating diseases caused by microbial infection,
preferably, an application of the granule of claim 8 in preparation of a drug for treating diseases caused by microbial infection;
preferably, an application of the pharmaceutical composition of claim 10 in preparation of a drug for treating diseases caused by microbial infection.

## Patentansprüche

1. Feste Dispersion eines Rifamycin-Nitroimidazol-Kopplungsmoleküls, umfassend ein Rifamycin-Nitroimidazol-Kopplungsmolekül einer in Formel I gezeigten Struktur, einen Polymerträger, einen funktionellen Hilfsstoff und ein Lösungsmittel,
wobei der Polymerträger eines oder eine Kombination aus mehreren von HPC-L, HPMC E3, Soluplus^{®} und Polymethacrylat umfasst;
wobei der funktionelle Hilfsstoff eines oder eine Kombination aus mehreren von Natriumlaurylsulfat, Meglumin, VE-TPGS und Tween 80 umfasst;

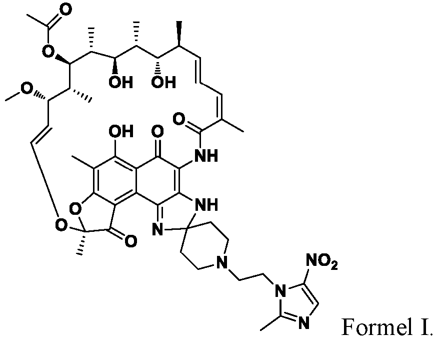

2. Feste Dispersion nach Anspruch 1, wobei das Polymethacrylat Eudragit^{®} EPO umfasst.

3. Feste Dispersion nach Anspruch 1, **dadurch gekennzeichnet, dass** die feste Dispersion durch eine oder mehrere XRPDs gekennzeichnet ist, die im Wesentlichen Fig. 8 ähnlich sind.

4. Feste Dispersion nach Anspruch 1, **dadurch gekennzeichnet, dass** die feste Dispersion durch ein oder mehrere Thermogramme gekennzeichnet ist, die im Wesentlichen Fig. 14-Fig. 19 ähnlich sind.

5. Feste Dispersion nach Anspruch 1, **dadurch gekennzeichnet, dass**, basierend auf der Gesamtmasse des Rifamycin-Nitroimidazol-Kopplungsmoleküls, des Polymerträgers und des funktionellen Hilfsstoffs als 100 %, die Verwendungsmenge des Rifamycin-Nitroimidazol-Kopplungsmoleküls 23,8%-71,2% beträgt, die Verwendungsmenge des Polymerträgers 23,8%-71,2% beträgt und die Verwendungsmenge des funktionellen Hilfsstoffs 3%-7% beträgt;
vorzugsweise der Polymerträger HPC-L und/oder Eudragit^{®} EPO umfasst;
vorzugsweise vorzugsweise der funktionelle Hilfsstoff VE-TPGS und/oder Tween 80 ist.

6. Feste Dispersion nach Anspruch 1, **dadurch gekennzeichnet, dass** das Lösungsmittel eines oder eine Kombination aus mehreren von Methanol, Ethanol, Aceton, Ethylacetat, Tetrahydrofuran und Dichlormethan umfasst.

7. Feste Dispersion nach Anspruch 1, **dadurch gekennzeichnet, dass**, wenn die feste Dispersion bei 40°C/75% RH oder 60°C/offenen Bedingungen für 4 Wochen gelagert wird, die feste Dispersion gemäß XRPD amorph bleibt.

8. Granulat, umfassend die feste Dispersion des Rifamycin-Nitroimidazol-Kopplungsmoleküls nach einem der Ansprüche 1-7.

9. Pharmazeutische Zusammensetzung, umfassend das Granulat nach Anspruch 8.

10. Pharmazeutische Zusammensetzung, umfassend die feste Dispersion des Rifamycin-Nitroimidazol-Kopplungsmoleküls nach einem der Ansprüche 1-7.

11. Anwendung der festen Dispersion des Rifamycin-Nitroimidazol-Kopplungsmoleküls nach einem der Ansprüche 1-7 bei der Herstellung eines Arzneimittels zur Behandlung von durch mikrobielle Infektionen verursachten Krankheiten,
vorzugsweise eine Anwendung des Granulats nach Anspruch 8 bei der Herstellung eines Arzneimittels zur Behandlung von durch mikrobielle Infektionen verursachten Krankheiten;
vorzugsweise eine Anwendung der pharmazeutischen Zusammensetzung nach Anspruch 10 bei der Herstellung eines Arzneimittels zur Behandlung von durch mikrobielle Infektionen verursachten Krankheiten.

## Revendications

1. Dispersion solide d'une molécule de couplage rifamycine-nitroimidazole, comprenant une molécule de couplage rifamycine-nitroimidazole d'une structure représentée par la formule I, un support polymère, un excipient fonctionnel et un solvant,
dans laquelle le support polymère comprend un ou une combinaison de plusieurs parmi HPC-L, HPMC E3, Soluplus^{®} et polyméthacrylate;
l'excipient fonctionnel comprend un ou une combinaison de plusieurs parmi le laurylsulfate de sodium, la méglumine, le VE-TPGS et le Tween 80;

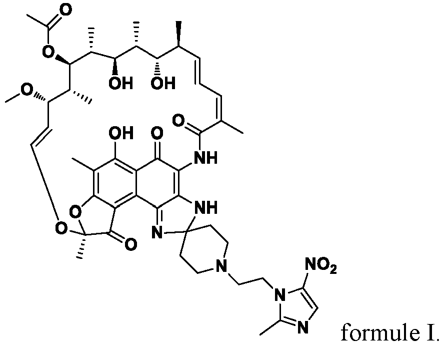

2. Dispersion solide selon la revendication 1, dans laquelle le polyméthacrylate comprend Eudragit^{®} EPO.

3. Dispersion solide selon la revendication 1, **caractérisée en ce que** la dispersion solide présente un ou plusieurs XRPD essentiellement similaires à Fig. 8.

4. Dispersion solide selon la revendication 1, **caractérisée en ce que** la dispersion solide présente un ou plusieurs thermogrammes essentiellement similaires à Fig. 14-Fig. 19.

5. Dispersion solide selon la revendication 1, **caractérisée en ce que**, sur la base de la masse totale de la molécule de couplage rifamycine-nitroimidazole, du support polymère et de l'excipient fonctionnel à 100 %, la proportion de la molécule de couplage rifamycin-nitroimidazole est de 23,8%-71,2 %, la proportion du support polymère est de 23,8%-71,2 %, et la proportion de l'excipient fonctionnel est de 3%-7 % ;
de préférence, le support polymère comprend HPC-L et/ou Eudragit^{®} EPO ;
de préférence, l'excipient fonctionnel est VE-TPGS et/ou Tween 80.

6. Dispersion solide selon la revendication 1, **caractérisée en ce que** le solvant comprend un ou une combinaison de plusieurs parmi le méthanol, l'éthanol, l'acétone, l'acétate d'éthyle, le tétrahydrofurane et le dichlorométhane.

7. Dispersion solide selon la revendication 1, **caractérisée en ce que**, lorsque la dispersion solide est placée à 40°C/75% HR ou 60°C/conditions ouvertes pendant 4 semaines, la dispersion solide reste amorphe selon le XRPD.

8. Granulé, comprenant la dispersion solide de la molécule de couplage rifamycine-nitroimidazole selon l'une quelconque des revendications 1 à 7.

9. Composition pharmaceutique, comprenant le granulé selon la revendication 8.

10. Composition pharmaceutique, comprenant la dispersion solide de la molécule de couplage rifamycine-nitroimidazole selon l'une quelconque des revendications 1 à 7.

11. Application de la dispersion solide de la molécule de couplage rifamycine-nitroimidazole selon l'une quelconque des revendications 1 à 7 dans la préparation d'un médicament pour le traitement de maladies causées par une infection microbienne,
de préférence, une application du granulé selon la revendication 8 dans la préparation d'un médicament pour le traitement de maladies causées par une infection microbienne;
de préférence, une application de la composition pharmaceutique selon la revendication 10 dans la préparation d'un médicament pour le traitement de maladies causées par une infection microbienne.
